# EUROPÄISCHE PATENTSCHRIFT

(11) **EP 1 931 765 B1**
(45) Veröffentlichungstag und Bekanntmachung des Hinweises auf die Patenterteilung: **17.08.2016**
(21) Anmeldenummer: 06791928.2
(22) Anmeldetag: 08.09.2006
(51) Int. Cl.: C12N 5/00

(54) **VERBESSERTES ZELLKULTURMEDIUM**
IMPROVED CELL CULTURE MEDIUM
MILIEU DE CULTURE CELLULAIRE AMELIORE

(30) Priorität: 28.09.2005 DE 102005046225
(43) Veröffentlichungstag der Anmeldung: 18.06.2008
(73) Patentinhaber: Cellca GmbH, 88471 Laupheim (DE)
(72) Erfinder: CAYLI, Aziz, 89075 Ulm (DE)
(74) Vertreter: Schrell, Andreas
(86) Internationale Anmeldenummer: PCT/EP2006/008761
(87) Internationale Veröffentlichungsnummer: WO 2007/036291

(56) Entgegenhaltungen:
- EP-A1- 1 227 155
- WO-A-2004/024899
- DE-A1- 2 946 898
- US-A1- 2003 059 935
- DATABASE WPI Week 199401 Derwent Publications Ltd., London, GB; AN 1994-002164 XP002433427 & JP 05 308962 A (JAPAN SYNTHETIC RUBBER CO LTD) 22. November 1993 (1993-11-22)

## Beschreibung

Die vorliegende Erfindung betrifft ein Verfahren zur Kultivierung mindestens einer Zelle und/oder zur Gewinnung eines Polypeptids, Proteins und/oder eines Impfstoffes aus Zellkulturen, wobei die Zellkulturen in einem Bernsteinsäure-haltigen Nährmedium kultiviert werden, und das gewünschte Polypeptid, Protein oder der Impfstoff aus den Organismen und/oder dem Kulturüberstand gewonnen wird. Die Erfindung betrifft auch die Verwendung eines Bernsteinsäure-haltigen Nährmediums zur Kultivierung von Säugetierzellen aus einer Zelllinie.

Es wurde überraschender Weise gefunden, dass die Zugabe von organischen Säuren oder Salzen, Derivaten oder Komplexen der organischen Säuren, ausgewählt aus der Gruppe Citronensäure, Bernsteinsäure, Äpfelsäure, α-Keto-Glutarsäure, Fumarsäure, Oxalessigsäure, Isocitronensäure, Oxalbernsteinsäure, Weinsäure, Adipinsäure, Milchsäure in ein Nährmedium das Wachstum und/oder die Produktivität der Zellen erhöht und/oder die toxischen Metabolitbildung reduziert hat. Die gemäß diesem Aspekt der Erfindung hervorgerufene Produktivitätssteigerung und/oder Reduzierung der Nebenproduktbildung kann erzielt werden, wenn die genannten Substanzen allein einem Nährmedium, insbesondere einem Zellkulturmedium, zugegeben werden, oder miteinander kombiniert werden, oder mit anderen Kohlenhydraten kombiniert werden, insbesondere wenn die Substanzen in ein Medium zugegeben werden, welches Glutaminarm ist, oder kein Glutamin enthält, oder mit einem Medium benutzt werden, welches Glutaminersatzstoffe enthält.

Die genannten Substanzen werden als Modulatoren des Zellstoffwechsels eingesetzt. Sie agieren als zusätzliche Energie- und/oder Kohlenhydratquelle. In diesem Zusammenhang können die Substanzen in einem Nährmedium, insbesondere in einem Zellkulturmedium, mit entsprechend höherer Konzentration verwendet werden, welches Kohlenhydrate und/oder Glutamin enthält. Die Substanzen können aber auch in einem Nährmedium, insbesondere in einem Zellkulturmedium, verwendet werden, welches keine Kohlenhydrate und/oder kein Glutamin enthält.

### Stand der Technik

Bei der Produktion von Impfstoffen, zum Beispiel Viren oder Polypeptiden, werden tierische Zellen, insbesondere Säugerzellen, zunehmend als Wirtszellen eingesetzt. Hier verwendet man meist kontinuierlich wachsende Zellen, also Zelllinien, welche entweder immortalisierte Zellen oder Tumorzellen sind. Für einige Anwendungen werden Primärzellen eingesetzt, die beispielsweise direkt aus Menschen oder Tieren entnommen werden. Es gibt große Unterschiede zwischen Primärzellen und Zelllinien, wenn sie in vitro kultiviert werden. Die Primärzellen können sich nur wenige Male teilen, während sich Zelllinien praktisch unendlich oft teilen können. Auch der Zellmetabolismus zwischen Primärzellen und kontinuierlichen Zelllinien kann unterschiedlich sein, beispielsweise die Glucoseverbrauchsrate, die Lactatbildungsrate und die Glutaminverbrauchsrate.

Ein Ziel der Prozessoptimierung bei der Verwendung von Zellen, insbesondere Säugerzellen ist, dass man versucht, das Integral der Lebendzellkonzentration im Kultivierungssystem zu erhöhen, also das Integral aus der Lebendzellkonzentrations- und der Zeitkurve.

Denn das Integral der Lebendzellkonzentration korreliert positiv mit der Produktkonzentration, zum Beispiel mit der Polypeptidkonzentration (Renard, J. M., et.al., Biotechnology Letters, 1988, 10(2): 91-96). Das Integral der Lebendzellkonzentration kann entweder durch die Erhöhung der Lebendzellkonzentration oder durch die Verlängerung der Prozesszeit erhöht werden.

Die Prozesszeit kann verlängert werden, wenn die Zellen lange im Bioreaktor am Leben bleiben, das heißt, wenn die Vitalität der Zellen lange hoch bleibt, beziehungsweise, wenn die stationäre Wachstumsphase verlängert wird. Ist die Vitalität niedrig, gibt es viele tote Zellen in der Kultur, die während der Fermentation lysieren und zelleigene Proteine freisetzen. Ein höherer Gesamt-Eigenproteingehalt in der Kultur erschwert wiederum die Aufreinigung des gewünschten Polypeptids. Für die Abnahme der Vitalität und somit kurze Prozesszeit gibt es mehrere Erklärungen: In einem Nährmedium, insbesondere in einem Zellkulturmedium, kann ein Substrat limitierend wirken, die Zellen können durch die physikalischen Fermentationsbedingungen in die Apoptose eintreten oder es können Stoffwechselnebenprodukte im Medium akkumulieren, die auf die Zellen toxisch wirken.

Wünschenswert wäre ein Nährmedium oder eine Methode, bei der die Zellvitalität lange hoch bleibt, das heißt bei der die stationäre Wachstumsphase verlängert wird, da in der stationären Wachstumsphase die höchste Lebendzellkonzentration schon erreicht ist. Die Verlängerung der stationären Wachstumsphase führt zu einer Erhöhung des Integrals der Lebendzellkonzentration und zu höherer Produktbildung. Ein anderer Parameter, um das Integral der Lebendzellkonzentration zu erhöhen, ist die Erhöhung der höchste erreichte Zellkonzentration (peak cell density). Durch bessere Nährmedien und durch bessere Kultivierungsverfahren kann die höchste erreichte Zellkonzentration weiter erhöht werden.

Die höchste erreichte Zellkonzentration oder Zellvitalität ist eine Funktion des Zellmetabolismus. Wenn die Zellen einen ineffizienten Metabolismus haben, scheiden sie energiereiche Zwischenmetabolite, wie Lactat ins Medium aus. Zellen können auch toxische Zwischenmetabolite ins Medium ausscheiden, wie Ammonium. Ammonium wiederum kann das Zellwachstum, Produktivität und Produktqualität, zum Beispiel Glykosilierung negativ beeinflussen. Zum Beispiel, wenn Zellen mit hoher glykolytischen Aktivität Glucose metabolisieren, wird die intrazelluläre Konzentration der glykolytischen Zwischenprodukte verschoben. Dies kann das Expressionsprofil diverser Gene beeinflussen, die für Stressstabilität, Produktivität und Wachstum notwendig sind (Verstrepen, K. J., et.al., Trends Biotechnol., 2004, 22 (10): 531-537). Unter solchen Bedingungen ist das Integral der Lebendzellkonzentration niedrig, was wiederum geringere Produktbildung bedeutet.

Bei kontinuierlichen Zelllinien ist bekannt, dass sie, im Vergleich zu Primärzellen, einen entarteten Zellmetabolismus haben. Eine Reihe von Enzymen des Primärmetabolismus zwischen Zelllinien und Primärzellen wurden in diesem Zusammenhang verglichen. In den geprüften Zelllinien konnte keine Aktivität von Enzymen, die die Glykolyse mit dem Citratzyklus verbinden, nachgewiesen werden, während sie in Primärzellen existieren (Neermann, J. und Wagner, R., J. Cell. Physiol., 1996, 166 (1): 152-169). In vielen kontinuierlichen Säugerzellen ist der Fluss von Glucose zu Lactat hoch, während der Fluss von Pyruvat zum Citratzyklus gering ist. Glucose wird durch die Zellen zu einem großen Teil zu Lactat umgewandelt und anschließend wird das energiereiche Lactat ins Nährmedium, insbesondere in ein Zellkulturmedium, ausgeschieden. Außer Glucose metabolisieren die Zelllinien mit hoher Rate Glutamin als Energiequelle. Somit gehören die beiden Substrate, Glucose und Glutamin zu den wichtigsten Substraten in Nährmedien für kontinuierliche Säugerzellen.

Es gab mehrere Ansätze, um den Zellmetabolismus der kontinuierlichen Zelllinien zu verbessern. Es wurde versucht, die Glucose und Glutamin im Prozess zu limitieren, um den glykolytischen Fluss zu bremsen. Durch die Substratlimitierung sollte der Overflow-Metabolismus der Zellen unterdrückt werden, somit sollten die Zellen weniger Lactat und Ammonium bilden (US 2004/0048368 A1; Europa, A.F., et.al., Biotechnol. Bioeng., 2000, 67(1): 25-34). Die Wirkung der Glutaminlimitierung wurde auf das Wachstum und die Apoptose der CHO-Zellen genau untersucht. Es wurde gezeigt, dass bei Glutaminlimitierung die Zellwachstumsrate abnimmt, aber der Anteil der Zellen, die in Apoptose eintreten geringer ist (Sanfeliu, A. und Stephanopoulus, G., Biotechnol. and Bioeng., 1999, 64(1): 46-53). In der WO 98/41611 wurde vorgeschlagen, die Glucose- und Glutaminkonzentration auf einen niedrigen Wert zu regeln. Dies soll durch die Kopplung der Glucose-, oder Glutaminkonzentration mit der Sauerstoffverbrauchsrate erfolgen. Diese Vorgehensweise setzt eine komplexe Regelung und Zufütterung der Substrate voraus, so dass der Prozess insbesondere unter Großmaßstabbedingungen schwer zu beherrschen ist. Das Risiko eines Absturzes des Fermenterlaufes und das damit verbundene finanzielle Risiko ist groß. Andererseits lehrt die EP 0435911 B1, dass unter anderem Glutamin in Standardzellkulturmedien erhöht werden muss, um verbessertes Zellwachstum und gesteigerte Produktbildung zu erzielen. In ähnlicher Weise sei die Zufütterung der tierischen Zellen mit Glutamin in Fed-batch oder in Perfusion für die Biopharmazeutikaproduktion notwendig (Duvar, S. et.al, Transkript, 2004, 5(1): 34-36). Insofern gibt es widersprüchliche Aussagen in der Literatur über die Rolle des Glutamins bezüglich des Zellstoffwechsels.

Chen, K., et.al. (Biotechnol. Bioeng., 2001, 72: 55-62) haben versucht, die Lactatbildung durch homologe Rekombination zu unterdrücken. Eine Kopie des Lactatdehydrogenase-Gens wurde inaktiviert und dadurch die zellspezifische Lactatbildungsrate 50 % reduziert. Irani, N., et.al. (J. Biotechnol., 1999, 66: 238-246) haben das Pyruvat-Carboxylase-Gen in eine Zelllinie kloniert. Die Erwartung war, dass die Zellen dadurch mehr Pyruvat in den Citratzyklus fördern und damit weniger Lactat produzieren. Gemäß der EP 0 338 841 A1 wurde das Glutaminsynthetase-Gen in Zellen als Selektionsmarker kloniert. Dies hatte den Vorteil, dass kein externes Glutamin ins Nährmediumzugegeben werden musste. Zellen haben selbst Glutamin produziert und wenig Ammonium ausgeschieden. Altamirano, C. et.al. (Biotechnol. Progress, 2000, 16(1): 69-75) haben in einem low-protein Medium unter Batch-Bedingungen Glucose und Glutamin durch schwer metabolisierbare Alternativen ersetzt, in diesem Falle mit Galactose und Glutamat. Dadurch haben die Zellen zwar weniger Stoffwechselnebenprodukte produziert, jedoch war sowohl das Zellwachstum als auch die Produktbildung niedriger als in der Kontrolle. Es wurde ferner vorgeschlagen einen Zweiphasenprozess zu entwickeln: Zellen sollen zuerst in Glucose wachsen und danach in der Produktionsphase Galactose als Kohlenhydratquelle verwerten (Altamirano, C., et.al., Biotechnol. Bioeng., 2001, 76(4): 351-60; Altamirano, C., et.al., J. Biotechnology, 2004, 110(2): 171-9). Die US 4,049,494 beschreibt ein serumfreies, chemisch definiertes und autoklavierbares Nährmedium. Das Medium besitzt die Eigenschaft, dass es glutaminfrei ist und gleichzeitig Glucose und Pyruvat enthält. Das Medium wurde speziell für die Virusproduktion mit Baby-Hamster-Kidney Zellen entwickelt. Ähnlich beschreibt WO 03/106661 A2 eine Substratkombination für die Kultivierung von Säugerzellen. Die Kombination umfasst ein glutaminfreies und gleichzeitig pyruvathaltiges Nährmedium. Diese Substratkombination soll die Bildung von Lactat und/oder Ammonium reduzieren. Es wurde gezeigt, dass eine Kombination von 1 mM Pyruvat mit 5,5 mM Glucose in einem Nährmedium die Ausreifung der Cumulus-cell-enclosed Maus Oocyten fördert. Interessanterweise konnte kein oder ein nur geringer Einfluss von Glutamin auf die Ausreifung der Zellen gezeigt werden (Downs, S.M. und Hudson, E. D., Zygote, 2000, 8(4): 339-51). Hassel, T. und Butler, M. (J. Cell Science, 1990, 96 (Pt3): 501-8) haben in einem Nährmedium Glutamin durch Glutamat oder 2-Oxoglutarat ersetzt und Zellen wurden in dieses Medium adaptiert. Nach der Adaptationsphase produzierten die verwendeten McCoy-Zellen weniger Lactat und Ammonium. Es ist zu betonen, dass die Zellen in diesem Ansatz auf Microcarriern wuchsen und sich entweder in der späteren exponentiellen Wachstumsphase oder in der stationären Wachstumsphase befanden. In diesen Wachstumsphasen haben die Zellen ein verändertes Metabolismusprofil. Außerdem war eine Voradaptierung der Zellen an glutaminfreies Medium notwendig.

In einem speziellen Nährmedium wurde Glutamin aus dem Nährmedium entfernt. Ohne Zelladaptation wuchs der untersuchte Klon einfach in dem speziellen Nährmedium. Dadurch wurde die Ammoniumbildung reduziert und Produktbildung erhöht (Deer, F., und Cunningham, M., Genetic Eng. News, 2000, 20(7): 42). Kurano, N., et.al., 1990 (Journal of Biotechnology, 15(1-2): 113-28) haben Glutamin durch Asparagin substituiert. Eine CHO-Kultur wurde unter fed-batch Bedingungen und unter glutaminfreien Bedingungen mit Asparagin supplementiert und dabei die Ammoniumbildung reduziert.

Glutamin wurde ferner in Nährmedien mit Dipeptiden, Alanyl-Glutamin (Ala-Gln) oder Glycyl-Glutamin (Gly-Gln) ersetzt. Es konnte gezeigt werden, dass durch diese Strategie die Zellen weniger Ammonium gebildet haben (Roth, E. et.al., In vitro cellular & developmental biology, 1988, 24(7): 696-698; Christie, A. und Butler, M., J. Biotechnology, 1994, 37(3):277-90). Glutamin wurde mit der stabilen Glutaminalternativen L-Alanyl-Glutamin (Glutamax) ersetzt um die Wirkung von Glutaminlimitierung mit dieser Substanz zu untersuchen (Sanfeliu, A. und Stephanopoulus, G., Biotechnol. und Bioeng., 1999, 64(1): 46-53).

In der EP 1 342 780 A1 wurde eine andere Stoffkombination veröffentlicht, welche den Glucoseverbrauch und die Lactatbildung reduzieren soll. Demnach soll dem Nährmedium freies ungebundens Citrat in Kombination mit in einem Komplexbildner gebundenem Eisencitrat zugegeben werden.

In einem Nährmedium (Perfusate) wurde Lactat und Pyruvat in einem Verhältnis 10:1 als Substrat zu den Ratten Herzzellen angeboten. Es wurde Lactat eingesetzt, weil Lactat ein bevorzugtes Substrat der Herzzellen ist. Es konnte gezeigt werden, dass der glykolytische Fluss durch Zugabe von Lactat inhibiert worden ist (Depre, C. et.al., Acta Cardiologica, 1993, 48(1): 147-64; Ovchinnikov, IV und Kim, N.P., Ukrainskii Biokhimicheskii Zhurnal, 1985, 57(4): 72-5).

In einem chemisch definierten proteinfreien Medium wurde die Wirkung von Succinat, Malat, Lactat und Pyruvat an Hamster-Embrionen untersucht. Es konnte gezeigt werden, dass Succinat und Malat in Nährmedium die Entwicklung der Blastocyten unterstützen (Ain, R. und Seshagiri, P.B., Molecular Reproduction & Development, 1997, 47(4): 440-7). In dieser Publikation war der Fokus auf die Entwicklungsbiologie gerichtet, speziell auf die Entwicklung der Blastocyten (Primärzellen).

Es wurde berichtet, dass die CHO (chinese hamster ovary) Zellen nicht in der Lage sind Ribose, Lactose, Sucrose, Glycerol, Lactat, Pyruvat, Citrat, Succinat, Fumarat oder Malat als Energiequelle zu verwerten (Faik, P. und Morgan, M.J., Cell biol. Int. Reports, 1977, 1(6): 555-62). Es wurde überraschender Weise herausgefunden, dass genau diese Substanzen durch Zellen, insbesondere durch CHO-Zellen, als Substrat verwendet werden können, wenn gemäß der Erfindung hier die richtige Konzentration gewählt wird und dabei die richtige Aminosäurekonzentration (Glutamin, Glutamat, Asparagin) und Kohlenhydratquelle gewählt wird.

Acetat wurde dem Nährmedium zugegeben, mit der Begründung, dass es die Produktivitätsleistung der Zellen in einer vergleichbaren Weise wie Butyrat erhöht (WO 03/064630). Glucose wurde mit einer anderen C2-Kohlenstoffquelle kombiniert um eine genetisch veränderte Hefestamm selektieren zu können (WO 2004/099425). In dieser Publikation ist der Fokus aber ein Verfahren zur Selektion eines neuen Hefestammes, nicht zur Kultivierung eines Organismus.

Die WO 2004/024899 A offenbart ein Citronensäure-haltiges Medium. Die US 2003/059935 A1 offenbart ein Milchsäure-haltiges Medium. Die DE 29 46898 A1 offenbart ein Bernsteinsäure-haltiges Medium. Die JP 05 308962 A offenbart ein Bernsteinsäure-haltiges Medium. Die US 5,508,191 A offenbart ein Bernsteinsäure-haltiges Medium zur Kultivierung von Bakterien. Die EP 1227155 offenbart ebenfalls ein Bernsteinsäure-haltiges Medium zur Kultivierung von Mikroorganismen.

### Technisches Problem

Das der Erfindung zugrunde liegende technische Problem ist die Bereitstellung eines Ausbeute-verbesserten Nährmediums, insbesondere eines Zellkulturmediums, einer Zellkultur und eines Verfahrens zur Kultivierung von Zellen beziehungsweise Zellkulturen, insbesondere zur Herstellung von biologischen Produkten aus den Zellen oder Zellkulturen, bei dem das Integral der Lebendzellkonzentration einer Zellkultur erhöht wird, und wobei dementsprechend über die Gesamtprozessdauer eine größere Menge an erwünschtem biologischen Produkt gewonnen werden kann. Wünschenswert ist es darüber hinaus auch, dass das Nährmedium, insbesondere das Zellkulturmedium, für Zellen eingesetzt werden kann, die einen oder mehr ggf. auch nicht genau lokalisierte Stoffwechselblockaden in Glykosylirungstoffwechselweg oder in Energiestoffwechselweg insbesondere im Citratzyklus oder Glykolyse aufweisen oder eine Blockade beim Transport von Metaboliten von Medium in die Zelle, und/oder in der Zelle zwischen den Organellen aufweisen.

Der Erfindung liegt daher auch das technische Problem zugrunde, ein Nährmedium zur Kultivierung von Zellen oder Zellkulturen, auch von Zellen mit Stoffwechselblockade in Glykosylierungsstoffwechselweg oder in Energiestoffwechselweg, z.B. im Citratzyklus oder Glykolyse, oder von Zellen, die eine Blockade in Transport von Metaboliten von Medium in die Zelle, und/oder in der Zelle zwischen den Organellen aufweisen bereitzustellen, bei dem das Zellwachstum verbessert wird, oder die Bildung der Stoffwechselnebenprodukte vermindert wird, wobei dementsprechend über die Gesamtprozessdauer eine größere Menge an erwünschtem biologischen Produkt gewonnen werden kann.

Der Erfindung liegt auch das technische Problem zugrunde, Verfahren zur Selektion spezieller Zellen, insbesondere genetisch modifizierter und/oder metabolisch veränderter, insbesondere optimierter, Zellen bereitzustellen.

Es wurde überraschender Weise gefunden, dass die Zugabe von organischen Säuren oder Salze, Derivate oder Komplexe der organischen Säuren, ausgewählt aus der Gruppe Citronensäure, Bernsteinsäure (erfindungsgemäß), Äpfelsäure, α-Keto-Glutarsäure, Fumarsäure, Oxalessigsäure, Isocitronensäure, Oxalbernsteinsäure, Weinsäure, Adipinsäure, Milchsäure in ein Nährmedium, insbesondere in ein Zellkulturmedium, das Wachstum und/oder die Produktivität der Zellen erhöht und/oder die toxischen Metabolitbildung reduziert hat. Erfindungsgemäß bevorzugt sind dabei organischen Säuren oder Salze oder Komplexe der organischen Säuren, ausgewählt aus der Gruppe Bernsteinsäure, Äpfelsäure, α-Keto-Glutarsäure, Fumarsäure, Oxalessigsäure, Isocitronensäure, Oxalbernsteinsäure, Weinsäure, Adipinsäure, Milchsäure.

Die gemäß diesem Aspekt hervorgerufene Produktivitätssteigerung und/oder Reduzierung der Nebenproduktbildung kann erzielt werden, wenn die erfindungsgemäßen Substanzen allein einem Nährmedium, insbesondere einem Zellkulturmedium, zugegeben werden, oder miteinander kombiniert werden, oder mit Kohlenhydraten kombiniert werden, oder ohne Kohlenhydrate ins Medium zugegeben werden, insbesondere wenn die Substanzen in ein Medium zugegeben werden, welches Glutamin enthält, oder kein Glutamin enthält, oder in einem Medium verwendet werden, welches Glutaminersatzstoffe enthält, oder keine Glutaminersatzstoffe enthält, oder eine hohe Asparaginkonzentration hat, oder Asparaginersatzstoffe enthält, oder eine hohe Glutamatkonzentration hat, oder Glutamatersatzstoffe enthält.

Die Erfindung löst das ihr zugrunde liegende technisches Problem insbesondere durch die Bereitstellung eines Verfahrens nach Anspruch 1 und einer Verwendung nach Anspruch 2..

Offenbart ist auch die Bereitstellung eines Nährmediums, insbesondere eines Zellkulturmediums, zur Kultivierung einer Zellkultur, umfassend mindestens eine Zelle, wobei das Nährmedium, insbesondere das Zellkulturmedium, mindestens eine Substanz, ausgewählt aus der Gruppe bestehend aus Citonensäure, Bernsteinsäure, Äpfelsäure, α-Keto-Glutarsäure, Fumarsäure, Oxalessigsäure, Isocitronensäure, Oxalbernsteinsäure, Weinsäure, Adipinsäure, Milchsäure und Mischungen davon sowie Salze, Derivate oder Komplexe dieser Säuren, enthält. Bevorzugt enthält das Nährmedium, insbesondere das Zellkulturmedium, mindestens eine Substanz, ausgewählt aus der Gruppe bestehend aus Bernsteinsäure, Äpfelsäure, α-Keto-Glutarsäure, Fumarsäure, Oxalessigsäure, Isocitronensäure, Oxalbernsteinsäure, Weinsäure, Adipinsäure, Milchsäure und Mischungen davon sowie Salze oder Komplexe dieser Säuren.

Unter Salzen der Säuren werden insbesondere Succinat (erfindungsgemäß), Malat, α-Keto-Glutarat, Fumarat, Oxalacetat, Iso-Citrat, Oxalsuccinat, Tartrat, Adipat, beziehungsweise Lactat verstanden. Unter dem Salz von Citronensäure wird Citrat verstanden.

Bevorzugt enthält das Nährmedium, insbesondere das Zellkulturmedium, mindestens zwei Substanzen, ausgewählt aus der Gruppe bestehend aus Bernsteinsäure, Äpfelsäure, α-Keto-Glutarsäure, Fumarsäure, Oxalessigsäure, Isocitronensäure, Oxalbernsteinsäure, Weinsäure, Adipinsäure, Milchsäure und Mischungen davon sowie Salze oder Komplexe dieser Säuren, wobei also eine oder mehrere der vorgenannten Säuren auch als Salz oder Komplex vorliegen können. Besonders bevorzugt ist die Gruppe um Citronensäure erweitert.

Bevorzugt enthält das Nährmedium Milchsäure. Bevorzugt enthält das Nährmedium Lactat. Bevorzugt enthält das Nährmedium mindestens einen Milchsäure-Komplex. Bevorzugt enthält das Nährmedium ein Milchsäure-Derivat.

Bevorzugt enthält das Nährmedium als Milchsäure-Derivat Alanin und/oder mindestens ein Alanin-haltiges Peptid. Bevorzugt enthält das Nährmedium Alanin und/oder mindestens ein Alanin-haltiges Peptid in einer Konzentration von 0,1 g/l oder größer, besonders bevorzugt von 1 g/l oder größer, mehr bevorzugt von 5 g/l oder größer, am meisten bevorzugt von 25 g/l oder größer. Bevorzugt enthält das Nährmedium als Milchsäure-Derivat Pyruvat und/oder mindestens ein Pyruvat-Derivat. Bevorzugt enthält das Nährmedium Pyruvat und/oder mindestens ein Pyruvat-Derivat in einer Konzentration von 0,1 g/l oder größer, besonders bevorzugt von 1 g/l oder größer, am bevorzugtesten von 5 g/l oder größer, am meisten bevorzugt von 25 g/l oder größer.

In einer bevorzugten Ausführungsform sind die in der Beschreibung aufgeführten Konzentrationen Endkonzentrationen. In einer bevorzugten Ausführungsform sind die in der Beschreibung aufgeführten Konzentrationen Gesamtkonzentrationen der jeweiligen Substanzen.

Bevorzugt enthält das Nährmedium Milchsäure, Lactat, mindestens einen Milchsäure-Komplex und/oder mindestens ein Milchsäure-Derivat in einer Konzentration von 0,1 g/l oder größer, bevorzugt von 1 g/l oder größer, besonders bevorzugt von 5 g/l oder größer, insbesondere bevorzugt von 25 g/l oder größer.

Erfindungsgemäß bevorzugt enthält das verwendete Nährmedium Bernsteinsäure. Erfindungsgemäß bevorzugt enthält das verwendete Nährmedium Succinat. Erfindungsgemäß bevorzugt enthält das verwendete Nährmedium ein Bernsteinsäure-Derivat. Erfindungsgemäß bevorzugt enthält das verwendete Nährmedium ein Succinat-Derivat.

In einer bevorzugten Ausführungsform der vorliegenden Erfindung weist das erfindungsgemäß verwendete Nährmedium, insbesondere das Zellkulturmedium, sämtliche der genannten Säuren oder Stoffe auf, wobei anstelle der Säuren auch die entsprechenden Salze oder Komplexe verwendet werden können. Insbesondere können in einer solchen Zusammensetzung stoffwechselblockierte Zellen kultiviert werden, insbesondere wenn der Ort des Blocks, z.B. in Glykosylierungsstoffwechselweg oder in Energiestoffwechselweg, beispielsweise im Citratzyklus oder Glykolyse nicht exakt lokalisierbar ist oder Zellen eine Blockade in Transport von Metaboliten von Medium in die Zelle, und/oder in der Zelle zwischen den Organellen aufweisen. Bevorzugt enthält das erfindungsgemäß verwendete Nährmedium, insbesondere das Zellkulturmedium, also Bernsteinsäure, Äpfelsäure, α-Keto-Glutarsäure, Fumarsäure, Oxalessigsäure, Isocitronensäure, Oxalbernsteinsäure, Weinsäure, Adipinsäure und Milchsäure, wobei eine oder mehrere der vorgenannten Säuren auch als Salz oder Komplex vorliegen können. Besonders bevorzugt ist die Gruppe um Citronensäure erweitert. Die offenbarten Substanzen sind zur dauerhaften Kultivierung der Organismen, besonders zur Kultivierung von tierischen Zellen, insbesondere zur Kultivierung von Säugerzellen, geeignet.

Bevorzugt enthält das erfindungsgemäß verwendete Nährmedium, insbesondere das erfindungsgemäße Zellkulturmedium, die mindestens eine offenbarte Substanz in einer Konzentration von mindestens 300 mg/l, am meisten bevorzugt mindestens 1000 mg/l. In einer weiteren bevorzugten Ausführungsform ist das erfindungsgemäße Nährmedium ein Zufütterungsmedium. Bevorzugt enthält das erfindungsgemäß verwendete Nährmedium, insbesondere Zufütterungsmedium, die mindestens eine erfindungsgemäße Substanz in einer Konzentration von 0,2 g/l oder größer, bevorzugter von 1 g/l oder größer, besonders bevorzugt von 5 g/l oder größer, sehr bevorzugt von 25 g/l oder größer, am meisten bevorzugt mindestens 100 g/l.

In einer bevorzugten Ausführungsform enthält das erfindungsgemäß verwendete Nährmedium, insbesondere das Zellkulturmedium, mindestens ein Kohlenhydrat, besonders bevorzugt ein Monosaccharid oder ein Disaccharid. Bevorzugt enthält das Nährmedium ein Kohlenhydrat. Bevorzugt ist das Kohlenhydrat ausgewählt aus der Gruppe bestehend aus Glucose, Galactose, Fructose, Mannose, Ribose, Glucosamin, Saccharose, Lactose und Mischungen davon.

Bevorzugt enthält das Nährmedium das mindestens eine Kohlenhydrat in einer Konzentration von 0,1 g/l oder größer, bevorzugter von 1 g/l oder größer, besonders bevorzugt von 5 g/l oder größer, insbesondere bevorzugt von 25 g/l oder größer, am meisten bevorzugt von 100 g/l oder größer.

Bevorzugt enthält das Nährmedium Galactose. Bevorzugt enthält das Nährmedium Galactose und ein zweites Kohlenhydrat. Bevorzugt enthält das Nährmedium Galactose in einer Konzentration von 0,1 g/l oder größer, bevorzugter von 1 g/l oder größer, besonders bevorzugt von 5 g/l oder größer, insbesondere bevorzugt von 25 g/l oder größer, am meisten bevorzugt von 100 g/l oder größer.

Bevorzugt enthält das Nährmedium Glucose in einer Konzentration von 100 g/l oder kleiner, bevorzugter 25 g/l oder kleiner, mehr bevorzugt 5 g/l oder kleiner, besonders bevorzugt von 1 g/l oder kleiner, insbesondere bevorzugt von 0,1 g/l oder kleiner. Bevorzugt ist das Nährmedium frei von Glucose.

In einer weiteren bevorzugten Ausführungsform ist das erfindungsgemäß verwendete Nährmedium, insbesondere das erfindungsgemäß verwendete Zellkulturmedium, frei von Kohlenhydraten.

Erfindungsgemäß enthält das erfindungsgemäß verwendete Nährmedium, insbesondere das Zellkulturmedium, Glutamin in einer Konzentration von kleiner als 8 mmol/l, bevorzugt von kleiner als 5 mmol/l, besonders bevorzugt von kleiner als 3 mmol/l, am bevorzugtesten von kleiner als 2 mmol/l. Das Nährmedium, insbesondere das Zellkulturmedium, kann aber auch bevorzugt frei von Glutamin sein.

In einer weiteren bevorzugten Alternative enthält das Nährmedium, insbesondere das Zellkulturmedium, mindestens ein Glutamin-Derivat, also mindestens einen Glutamin-haltigen Glutaminersatzstoff, besonders bevorzugt ein Glutamin-haltiges Dipeptid. Bevorzugt ist das mindestens eine Gluta-min-Derivat ein Glutamin-haltiges Peptid.

Bevorzugt enthält das Nährmedium, insbesondere das Zellkulturmedium, Glutamin-Derivate in einer Gesamt-Konzentration von größer als 30 mg/l, bevorzugt größer als 150 mg/l, besonders bevorzugt größer als 750 mg/l, am bevorzugtesten größer als 2000 mg/l. Bevorzugt enthält das Nährmedium, insbesondere das Zellkulturmedium, Glutamin-Derivate in einer Konzentration von 0,05 g/l oder größer, bevorzugt von 0,2 g/l oder größer, besonders bevorzugt von 0,8 g/l oder größer, am bevorzugtesten von 3,2 g/l oder größer. Das Nährmedium, insbesondere das Zellkulturmedium, kann aber auch in einer bevorzugten Alternative frei von Glutamin-Derivaten sein.

Bevorzugt enthält das Nährmedium Asparagin. Bevorzugt enthält das Nährmedium mindestens ein Asparagin-Ersatzstoff, insbesondere mindestens ein Asparagin-Derivat. Bevorzugt enthält das Nährmedium, insbesondere das Zellkulturmedium, Asparagin oder mindestens einen Asparaginersatzstoff, besonders bevorzugt mindestens ein Asparagin-Derivat, in einer hohen Konzentration, das heißt in einer Konzentration, insbesondere Gesamtkonzentration, von größer als 0,05 g/l, stark bevorzugt von größer als 0,15 g/l, mehr bevorzugt von größer als 0,3 g/l, insbesondere von größer als 0,6 g/l

Bevorzugt enthält das Nährmedium, insbesondere das Zellkulturmedium, Glutamat oder mindestens einen Glutamatersatzstoff, bevorzugt mindestens eine Glutamat-Derivat, in einer hohen Konzentration, das heißt in einer Konzentration von 0,05 g/l oder größer, bevorzugter von 0,15 g/l oder größer, mehr bevorzugt von 0,3 g/l oder größer, insbesondere von 0,6 g/l oder größer.

Offenbart ist ein Zellkulturmedium, das Milchsäure und Galactose sowie die vorgenannte hohe Aspargin-Konzentration enthält, gleichzeitig aber frei von Glutamin ist.

Offenbart ist ein Zellkulturmedium, welches Milchsäure, Galactose und Aspargin in der vorgenannten hohen Konzentration enthält sowie gleichzeitig frei von Glutamin ist, aber zumindest ein Glutamin-haltiges Peptid aufweist.

In einer weiteren bevorzugten Ausführungsform offenbart die vorliegende Beschreibungdie Verwendung eines Zellkulturmediums, welches Milchsäure und Galactose aufweist, darüber hinaus eine hohe Glutamat-Konzentration in der vorgenannten Konzentration aufweist, gleichzeitig aber frei von Glutamin ist.

In einer weiteren bevorzugten Ausführungsform betrifft die vorliegende Erfindung die Verwendung Zellkulturmediums, welches Bernsteinsäure und eine hohe Aspargin- oder Aspargin-Ersatzstoffkonzentration der vorgenannten Konzentrationen aufweist, gleichzeitig aber frei von Glutamin ist.

Bevorzugt ist das Nährmedium frei von Glucose und frei von Glutamin und enthält Asparagin in einer Konzentration von 0,05 g/l oder größer, bevorzugter von 0,15 g/l oder größer, besonders bevorzugt von 0,3 g/l oder größer, am bevorzugtesten von 0,6 g/l oder größer, sowie Milchsäure und Galactose. Bevorzugt ist das Nährmedium frei von Glucose und Glutamin ist und enthält Glutamat in einer Konzentration von 0,05 g/l oder größer, bevorzugter von 0,15 g/l oder größer, besonders bevorzugt von 0,3 g/l oder größer, noch mehr bevorzugt von 0,6 g/l oder größer, sowie Milchsäure und Galactose. Bevorzugt ist das Nährmedium frei von Glucose ist und enthält Glutamin-haltige Peptide, Milchsäure und Galactose.

In einer weiteren bevorzugten Ausführungsform ist das erfindungsgemäß verwendete Nährmedium, insbesondere das erfindungsgemäße Zellkulturmedium, ein Zufütterungsmedium.

Bevorzugt enthält das Zufütterungsmedium Milchsäure, Lactat, mindestens einen Milchsäure-Komplex und/oder mindestens ein Milchsäure-Derivat in einer Konzentration von 1 g/l oder größer, bevorzugt von 5 g/l oder größer, besonders bevorzugt von 25 g/l oder größer, insbesondere bevorzugt von 100 g/l oder größer.

Bevorzugt enthält das Zufütterungsmedium Galactose in einer Konzentration von 1 g/l oder größer, besonders bevorzugt von 5 g/l oder größer, insbesondere bevorzugt von 25 g/l oder größer, am meisten bevorzugt von 100 g/l oder größer.

Bevorzugt enthält das Nährmedium, besonders bevorzugt Zufütterungsmedium, Glutamin-Derivate in einer Konzentration von von 0,2 g/l oder größer, mehr bevorzugt von 1 g/l oder größer, besonders bevorzugt von 5 g/l oder größer, am bevorzugtesten von 10 g/l oder größer.

Bevorzugt enthält das Nährmedium, insbesondere das Zufütterungsmedium, Asparagin oder mindestens einen Asparaginersatzstoff, besonders bevorzugt mindestens ein Asparagin-Derivat, in einer Konzentration von 0,2 g/l oder größer, mehr bevorzugt von 1 g/l oder größer, besonders bevorzugt von 2 g/l oder größer, insbesondere von 5 g/l oder größer, am bevorzugtesten von 10 g/l oder größer.

Bevorzugt enthält das Zufütterungsmedium Glutamat oder mindestens einen Glutamatersatzstoff, besonders bevorzugt mindestens ein Glutamat-Derivat, in einer Konzentration von 0,2 g/l oder größer, bevorzugt von 1 g/l oder größer, besonders bevorzugt von 2 g/l oder größer, insbesondere von 5 g/l oder größer, am bevorzugtesten von 10 g/l oder größer.

In einer bevorzugten Alternative ist das erfindungsgemäß verwendete Nährmedium, insbesondere das erfindungsgemäß verwendete Zellkulturmedium, ein Basalmedium, bevorzugt ein Basalmedium mit einer Osmolalität von 240 bis 360 mOsmol/kg H₂O. In einer bevorzugten Alternative ist das erfindungsgemäß verwendete Nährmedium, insbesondere das erfindungsgemäß verwendete Zellkulturmedium, ein Basalmedium, bevorzugter ein Basalmedium mit einer Osmolalität von 280 bis 350 mOsmol/kg H₂O, am meisten bevorzugt ein Basalmedium mit einer Osmolalität von 280 bis 320 mOsmol/kg H₂O.

In einer weiteren bevorzugten Alternative ist das Nährmedium, insbesondere das Zellkulturmedium, ein Zufütterungsmedium (auch Feedmedium genannt), bevorzugt ein Zufütterungsmedium mit einer Osmolalität von 150 bis 1500 mOsmol/kg H₂O.

Bevorzugt ist das Nährmedium Serum-frei. Bevorzugt enthält das Nährmedium Serum.

Ein Nährmedium ist insbesondere ein Zellkulturmedium. Bevorzugt ist das Nährmedium ein flüssiges Nährmedium, insbesondere ein flüssiges Zellkulturmedium.

Für einen Fachmann sind durch die angegebenen bevorzugten Mindestangaben für die Konzentration der genannten Inhaltsstoffe im Nährmedium die zu wählenden Konzentrationsbereiche der Inhaltsstoffe, also auch die zu wählenden Höchstkonzentrationen der Inhaltsstoffe, ohne besonderen Aufwand zu ermitteln. Bevorzugt beträgt die Konzentration einer eingesetzten organischen Säure höchstens 200 g/l, besonders bevorzugt 100 g/l, mehr bevorzugt 50 g/l, am meisten bevorzugt 25 g/l. Bevorzugt beträgt die Konzentration von eingesetztem Lactat und/oder von einem Milchsäure-Derivat, insbesondere Alanin und/oder Pyruvat, höchstens 200 g/l, besonders bevorzugt 100 g/l, insbesondere 50 g/l, am meisten bevorzugt 25 g/l. Bevorzugt beträgt die Konzentration eines eingesetzten Glutamin-Derivats höchstens 60 g/l, besonders bevorzugt 30 g/l, mehr bevorzugt 10 g/l, sehr bevorzugt 5 g/l, insbesondere 1,2 g/l, am meisten bevorzugt 1,1 g/l. Bevorzugt beträgt die Konzentration von eingesetztem Asparagin oder eines Asparagin-Derivats höchstens 60 g/l, besonders bevorzugt 30 g/l, mehr bevorzugt 5 g/l, insbesondere 1,2 g/l, am meisten bevorzugt 1,1 g/l. Bevorzugt beträgt die Konzentration von eingesetztem Glutamat oder eines Glutamat-Derivats höchstens 60 g/l, besonders bevorzugt 30 g/l, mehr bevorzugt 5 g/l, insbesondere 1,2 g/l, am meisten bevorzugt 1,1 g/l. Bevorzugt beträgt die Konzentration von eingesetzter Galactose höchstens 200 g/l, besonders bevorzugt 100 g/l, insbesondere 50 g/l, am meisten bevorzugt 25 g/l.

Die Erfindung betrifft auch die Verwendung eines Nährmediums, insbesondere eines Zellkulturmediums, zum Kultivieren einer Zellkultur, umfassend mindestens eine Zelle, wie in Anspruch 2 dargelegt.

Die Erfindung offenbart auch die Bereitstellung einer Zelle oder einer Zellkultur. Die Zelle oder Zellkultur ist bevorzugt metabolisch soweit optimiert, dass sie in hier offenbarten Kulturmedien und erfindungsgemäßen Verfahren kultiviert werden kann.

Die Zelle oder Zellkultur ist bevorzugt dadurch gekennzeichnet, dass die Zellkultur in einem Medium mit hier offenbarten Substanzen überleben kann oder teilen kann oder die höchste erreichte Zellkonzentration (peak cell density) höher ist, oder die stationäre Wachstumsphase verlängert ist oder die Absterbephase verlangsamt ist, oder die Bildung der Zell-metabolischen Nebenprodukte, zum Beispiel Ammonium oder Lactat reduziert ist.

Bevorzugt ist eine Zelle oder Zellkultur, welche Milchsäure, sowie Salze (Lactat) oder Komplexe der Milchsäure-, oder Salze von Milchsäure-Ersatzstoffen metabolisieren kann. Bevorzugt ist eine Zelle oder Zellkultur, welche in einem glucosefreien und glutamin-freien Medium überleben kann, bevorzugt teilen kann, besonders bevorzugt höhere integral of viable cells (IVC) erreichen kann, am bevorzugtesten geringer Stoffwechselnebenprodukte, insbesondere Lactat oder Ammonium produziert. Bevorzugt ist eine Zelle oder Zellkultur, welche in einem Glucose-freien Medium überleben kann, bevorzugt teilen kann, besonders bevorzugt höhere integral of viable cells (IVC) erreichen kann, am bevorzugtesten geringer Stoffwechselnebenprodukte, insbesondere Lactat oder Ammonium produziert. Bevorzugt ist eine Zelle oder Zellkultur, welche in einem Glucose-freien und Glutamin-freien Medium überleben kann, und Lactat metabolisieren kann, bevorzugt teilen kann, besonders bevorzugt höhere integral of viable cells (IVC) erreichen kann, am bevorzugtesten geringer Stoffwechselnebenprodukte, insbesondere Lactat oder Ammonium produziert. Bevorzugt ist eine Zelle oder Zellkultur, welche in einem Glucose-freien und Glutamin-freien Medium überleben kann, und Lactat metabolisieren kann und einen hohen Asparaginverbrauch hat, sich in einem solchen Medium bevorzugt teilen kann, besonders bevorzugt höhere integral of viable cells (IVC) erreichen kann, am bevorzugtesten geringer Stoffwechselnebenprodukte, insbesondere Lactat oder Ammonium produziert. Bevorzugt ist eine Zelle oder Zellkultur, welche in einem Glucose-freien und Glutamin-freien Medium überleben kann, und Lactat metabolisieren kann und einen hohen Glutamatverbrauch hat, sich in einem solchen Medium bevorzugt teilen kann, besonders bevorzugt höhere integral of viable cells (IVC) erreichen kann, am bevorzugtesten geringer Stoffwechselnebenprodukte, insbesondere Lactat oder Ammonium produziert. Bevorzugt ist eine Zelle oder Zellkultur, welche in einem Glucose-freien und Glutamin-freien Medium überleben kann, und Lactat metabolisieren kann und Galactose metabolisieren kann, sich in einem solchen Medium bevorzugt teilen kann, besonders bevorzugt höhere integral of viable cells (IVC) erreichen kann, am bevorzugtesten geringer Stoffwechselnebenprodukte, insbesondere Lactat oder Ammonium produziert. Bevorzugt ist eine Zelle oder Zellkultur, welche in einem Glucose-freien und Glutamin-freien Medium überleben kann, und Lactat metabolisieren kann und Galactose metabolisieren kann, und einen hohen Asparaginverbrauch oder einen hohen Glutamatverbrauch hat, sich in einem solchen Medium bevorzugt teilen kann, besonders bevorzugt höhere integral of viable cells (IVC) erreichen kann, am bevorzugtesten geringer Stoffwechselnebenprodukte, insbesondere Lactat oder Ammonium produziert.

Offenbart ist auch die Bereitstellung eines Verfahrens zum Kultivieren einer Zellkultur, umfassend mindestens eine Zelle, wobei die Zellkultur in einem Nährmedium eingebracht und kultiviert wird und wobei der pH-Wert des Nährmediums während mindestens eines Fünftels der Kultivierungszeitdauer pH 7,2 oder basischer ist.

Die Erfindung löst das ihr zugrunde liegende technische Problem auch durch die Bereitstellung eines Verfahrens nach Anspruch 1 zum Kultivieren einer Zellkultur, umfassend mindestens eine Zelle, wobei die Zellkultur in einem Nährmedium nach Anspruch 1, insbesondere in einem erfindungsgemäßen Zellkulturmedium, eingebracht und kultiviert wird.

Bevorzugt wird die in das Nährmedium, insbesondere in das Zellkulturmedium, eingebrachte Zellkultur, umfassend mindestens eine Zelle, zur Herstellung eines biologischen Produktes verwendet.

Bevorzugt ist das biologische Produkt ausgewählt aus mindestens einem Polypeptid, mindestens einem Protein, mindestens einem Virusbestandteil, mindestens einem Virus oder aus einer Mischung aus diesen. Besonders bevorzugt ist das biologische Produkt mindestens ein Impfstoff.

Bevorzugt wird das biologische Produkt aus der Zellkultur und/oder aus dem Nährmedium, insbesondere aus dem erfindungsgemäßen Zellkulturmedium, gewonnen.

Bevorzugt enthält die mindestens eine Zelle mindestens ein Gen, das das biologische Produkt codiert.

Erfindungsgemäß wird unter Zellkultur mindestens eine Zelle verstanden, die in einem Medium, bevorzugt einem Nährmedium, insbesondere einem Zellkulturmedium, am Leben erhalten wird, sich bevorzugt auch in dem Medium teilt beziehungsweise zur Zellteilung angeregt wird. Insbesondere ist die mindestens eine Zelle eine eukaryontische Zelle, besonders bevorzugt eine Pilz-Zelle, insbesondere eine Hefe-Zelle, eine pflanzliche Zelle oder eine tierische Zelle, beispielsweise eine Insekten-Zelle oder erfindungsgemäß eine Säugetier-Zelle, insbesondere eine humane oder Nagerzelle, eine Hamsterzelle oder eine Myelomazelle, zum Beispiel eine CHO-, NS0-, PERC6-, HEK-293- oder BHK21-Zelle. Die mindestens eine Zelle kann auch eine prokaryotische Zelle sein. Bevorzugt ist die mindestens eine Zelle ein Abkömmling der einer der obengenannten Zellen.

Erfindungsgemäß stammt die mindestens eine Zelle aus einer Zelllinie, insbesondere einer immortalisierten Zelllinie und/oder einer Tumor-Zelllinie. Ebenfalls bevorzugt ist die mindestens eine Zelle eine Primärzelle. Beispiele für erfindungsgemäße Zellen sind im weiteren Verlauf des Textes aufgeführt.

Die Zellkultur ist erfindungsgemäß bevorzugt eine Zellpopulation. Die Zellkultur ist erfindungsgemäß bevorzugt alternativ ein Zellklon. Die Zellkultur kann erfindungsgemäß auch mehrere Zelltypen oder Zellarten umfassen, bevorzugt kann die Zellkultur aus mehreren Zelltypen oder Zellarten bestehen beziehungsweise diese umfassen. Die Zellkultur kann erfindungsgemäß bevorzugt aus Zellverbänden bestehen oder Zellverbände umfassen.

Erfindungsgemäß bevorzugt handelt es sich bei dem erfindungsgemäßen Verfahren um ein Batch-Verfahren, um ein Split-Batch-Verfahren, um ein Fed-Batch-Verfahren, um ein kontinuierliches Verfahren oder um ein Perfusionsverfahren.

Erfindungsgemäß bevorzugt beträgt der pH-Wert des Nährmediums bei Beendigung des Kultivierens 7,2 oder basischer, mehr bevorzugt 7,3 oder basischer, besonders bevorzugt 7,4 oder basischer, insbesondere bevorzugt 7,5 oder basischer, ganz besonders bevorzugt 7,6 oder basischer, am meisten bevorzugt 7,7 oder basischer.

Erfindungsgemäß bevorzugt beträgt der pH-Wert des Nährmediums während mindestens eines Fünftels, mehr bevorzugt während mindestens zwei Fünftel, insbesondere bevorzugt während mindestens drei Fünftel, insbesondere während mindestens vier Fünftel, der Kultivierungsdauer pH 7,2 oder basischer" mehr bevorzugt 7,3 oder basischer, besonders bevorzugt 7,4 oder basischer, insbesondere bevorzugt 7,5 oder basischer, ganz besonders bevorzugt 7,6 oder basischer, am meisten bevorzugt 7,7 oder basischer.

Erfindungsgemäß bevorzugt beträgt der pH-Wert des Nährmediums während des Kultivierungsprozesses pH 7,2 oder basischer, mehr bevorzugt 7,3 oder basischer, besonders bevorzugt 7,4 oder basischer, insbesondere bevorzugt 7,5 oder basischer, ganz besonders bevorzugt 7,6 oder basischer, am meisten bevorzugt 7,7 oder basischer.

Erfindungsgemäß bevorzugt beträgt der pH-Wert des Nährmediums während des Kultivierungsprozesses mindestens pH 6,6 und höchstens pH 7,9 , mehr bevorzugt mindestens pH 6,7 und höchstens pH 7,9, am meisten bevorzugt mindestens pH 6,8 und höchstens pH 7,9. Erfindungsgemäß bevorzugt beträgt der pH-Wert des Nährmediums während des Kultivierungsprozesses mindestens pH 6,6 und höchstens 7,5 , mehr bevorzugt mindestens pH 6,7 und höchstens pH 7,5, am meisten bevorzugt mindestens pH 6,8 und höchstens pH 7,5. Erfindungsgemäß bevorzugt beträgt der pH-Wert des Nährmediums während des Kultivierungsprozesses mindestens pH 6,6 und höchstens 7,2 , mehr bevorzugt mindestens pH 6,7 und höchstens pH 7,2, am meisten bevorzugt mindestens pH 6,8 und höchstens pH 7,2.

Erfindungsgemäß bevorzugt ist der pH Wert des Nährmediums während des Kultivierungsprozesses nie höher als pH 9,0, sehr bevorzugt nie höher als pH 8,5, mehr bevorzugt nie höher als pH 8,0, insbesondere bevorzugt nie höher als pH 7,8, am meisten bevorzugt nie höher als pH 7,7.

Erfindungsgemäß bevorzugt beträgt der pH-Wert während des Kultivierungsprozesses des erfindungsgemäßen Verfahrens basischer als pH 7,2, bevorzugter basischer als pH 7,3, mehr bevorzugt basischer als pH 7,4, bevorzugter basischer als pH 7,5, insbesondere bevorzugt basischer als pH 7,6, am bevorzugtesten basischer als pH 7,7. Erfindungsgemäß bevorzugt beträgt der pH-Wert während des Kultivierungsprozesses des erfindungsgemäßen Verfahrens mindestens in vier fünftel (4/5) der Gesamtkultivierungszeit des erfindungsgemäßen Verfahrens basischer als pH 7,2, bevorzugt basischer als pH 7,3, mehr bevorzugt basischer als pH 7,4 bevorzugter basischer als pH 7,5, insbesondere bevorzugt basischer als pH 7,6, am bevorzugtesten basischer als pH 7,7. Wobei Gesamtkultivierungszeit des erfindungsgemäßen Verfahrens startet wenn ein Kulturgefäß mit Zellen in der Produktionsstufe angeimpft wird. Also die Zwischenstufen einer Zellkultivierung um Zellen für die Produktionsstufe zu expandieren zählt nicht zur Gesamtkultivierungszeit.

Erfindungsgemäß bevorzugt beträgt der pH-Wert am Ende des Kultivierungsprozesses des erfindungsgemäßen Verfahrens basischer als pH 7,2, bevorzugt basischer als pH 7,3, mehr bevorzugt basischer als pH 7,4 bevorzugter basischer als pH 7,5, insbesondere bevorzugt basischer als pH 7,6, am bevorzugtesten basischer als pH 7,7. Erfindungsgemäß bevorzugt beträgt der pH Wert des erfindungsgemäßen Verfahrens höchsten 7,8 mehr bevorzugt höchstens 8,0.

Die Beschreibung offenbart auch ein Verfahren zur Herstellung eines hier offenbarten Nährmediums, insbesondere eines Zellkulturmediums, wobei mindestens eine Substanz, ausgewählt aus der Gruppe bestehend aus Citronensäure, Bernsteinsäure, Äpfelsäure, α-Keto-Glutarsäure, Fumarsäure, Oxalessigsäure, Isocitronensäure, Oxalbernsteinsäure, Weinsäure, Adipinsäure, Milchsäure und Mischungen davon sowie Salze, Derivate oder Komplexe dieser Säuren, in Wasser oder einem Zellkulturlösemittel beziehungsweise Nährmediumlösemittel, insbesondere einem Zellkulturmediumlösemittel, oder einem herkömmlichen Nährmedium, insbesondere Zellkulturmedium, gelöst wird beziehungsweise werden.

Die Beschreibung offenbart auch ein Verfahren, insbesondere ein erfindungsgemäßes Verfahren zum Kultivieren einer Zellkultur, zur Selektion einer genetisch modifizierten, insbesondere metabolisch optimierten, Zelle aus einer Zellpopulation, wobei a) eine Zellkultur als Zellpopulation in einem erfindungsgemäßen Nährmedium für mindestens eine Woche kultiviert wird, besonders bevorzugt von mindestens vier Wochen kultiviert wird; und anschließend b) die Zelle isoliert wird. Die isolierte Zelle ist eine lebende Zelle. Bevorzugt findet die Kultivierung gemäß einer erfindungsgemäßen Kultivierungsmethode statt.

Es zeigte sich überraschender Weise, dass sich bei Kultivierung in einem offenbarten Nährmedium neuartige Zellen selektieren und isolieren lassen, die genetisch modifiziert sind. Die genetische Modifikation zeigt sich beispielsweise durch eine Veränderung, insbesondere Verbesserung des Zellmetabolismus. Insbesondere solche Zellen mit einem solchen veränderten Zellmetabolismus wachsen im vergleich zu herkömmlichen Zellen besonders gut, beziehungsweise überhaupt, in einem erfindungsgemäßen Medium. Der veränderte Zellmetabolismus entsteht durch genetische Modifikationen. Somit lassen sich durch Kultivierung von Zellpopulationen in einem erfindungsgemäßen Nährmedium solche Zellen isolieren, die genetisch modifiziert sind.

Bevorzugt wird die mindestens eine in Schritt b) isolierte Zelle in einem weiteren Schritt c) in ein neues Kulturmedium überführt. Erfindungsgemäß besonders bevorzugt ist das neue Nährmedium ein frisches Nährmedium. Besonders bevorzugt ist das neue Nährmedium ein hier offenbartes Nährmedium. Bevorzugt wird die mindestens eine in Schritt b) isolierte Zelle in Schritt c) expandiert.

Erfindungsgemäß bevorzugt hat das Nährmedium bei der Durchführung des erfindungsgemäßen Verfahrens einen pH Wert von basischer als 7,2, bevorzugt basischer als pH 7,3, noch mehr bevorzugt basischer als pH 7,4, bevorzugter basischer als pH 7,5, insbesondere bevorzugt basischer als pH 7,6, am bevorzugtesten basischer als pH 7,7.

Erfindungsgemäß bevorzugt hat das Nährmedium bei der Durchführung des erfindungsgemäßen Verfahrens mindestens in vier fünftel (4/5) der Gesamtkultivierungszeit einen pH-Wert basischer als pH 7,2, bevorzugt basischer als pH 7,3, mehr bevorzugt basischer als pH 7,4 bevorzugter basischer als pH 7,5, insbesondere bevorzugt basischer als pH 7,6, am bevorzugtesten basischer als pH 7,7. Die Gesamtkultivierungszeit des erfindungsgemäßen Verfahrens startet wenn ein Kulturgefäß mit Zellen in der Produktionsstufe angeimpft wird. Also die Zwischenstufen einer Zellkultivierung um Zellen für die Produktionsstufe zu expandieren zählt nicht zur Gesamtkultivierungszeit.

Erfindungsgemäß bevorzugt hat das Nährmedium am Ende des Kultivierungsprozesses einen pH Wert von basischer als 7,2, bevorzugt basischer als pH 7,3, noch mehr bevorzugt basischer als pH 7,4, bevorzugter basischer als pH 7,5, insbesondere bevorzugt basischer als pH 7,6, am bevorzugtesten basischer als pH 7,7. Erfindungsgemäß bevorzugt beträgt der pH Wert höchstens 7,8 mehr bevorzugt höchstens 8,0.

Erfindungsgemäß bevorzugt wird die mindestens eine Zelle mindestens eine Passage, besonders bevorzugt mindestens 2 Passagen, am meisten bevorzugt mindestens 5 Passagen lang subkultiviert. Erfindungsgemäß bevorzugt wird die mindestens eine Zelle so lang subkultiviert wird, bis ein Splitverhältnis von größer 1 zu 4 erreicht wird.

Offenbart ist auch eine selektierte Zelle, erhältlich aus einem hier offenbarten Verfahren. Bevorzugt kann die Zelle Lactat als Kohlenstoffquelle metabolisieren. Bevorzugt besitzt die Zelle ein Splitverhältnis von mindestens 1 zu 4 mit Galactose als einziger Kohlenstoffquelle. Bevorzugt besitzt die Zelle in einem Glucose- und Glutamin-freien Medium ein Splitverhältnis von mindestens 1 zu 4. Bevorzugt beträgt die Wachstumsrate (µ) der Zelle weniger als 24 Stunden, besonders bevorzugt weniger als 20 Stunden, besonders bevorzugt weniger als 18 Stunden. Offenbart ist auch eine Zellkultur, die mindestens eine erfindungsgemäße Zelle enthält.

Bevorzugt kann die Zellkultur Lactat als Kohlenstoffquelle metabolisieren. Bevorzugt besitzt die Zellkultur ein Splitverhältnis von mindestens 1 zu 4 mit Galactose als einziger Kohlenstoffquelle. Bevorzugt besitzt die Zellkultur in einem Glucose- und Glutamin-freien Medium ein Splitverhältnis von mindestens 1 zu 4. Bevorzugt beträgt die Wachstumsrate (µ) der so isolierten Zellkultur weniger als 24 Stunden, besonders bevorzugt weniger als 20 Stunden, besonders bevorzugt weniger als 18 Stunden.

Erfindungsgemäß ist auch ein Verfahren zur Gewinnung eines biologischen Produkts aus mindestens einer Zelle, wobei die mindestens eine Zelle gemäß einem erfindungsgemäßen Verfahren kultiviert und daraus das biologische Produkt gewonnen wird.

Die gemäß diesem Aspekt offenbarten Substanzen sind insbesondere Bernsteinsäure, Apfelsäure, α-Keto-Glutarsäure, Fumarsäure, Oxalessigsäure, Isocitronensäure, Oxalbernsteinsäure, Weinsäure, Adipinsäure und Milchsäure. Sehr bevorzugt kann zu diesen Substanzen auch Citronensäure gehören.

Die gemäß diesem Aspekt offenbarten notwendigen Substanzen können insbesondere einzeln in einem Nährmedium, insbesondere in einem Zellkulturmedium, addiert werden, vorzugsweise werden sie, das heißt einige oder alle miteinander kombiniert. Bevorzugt ist aber auch die Kombination mit anderen Kohlenhydraten. Die geeigneten Kohlenhydrate sind vorzugsweise aus Mono- und Disacchariden wie etwa Glucose, Galactose, Glucosamin, Fructose, Ribose, Mannose, Saccharose, Lactose ausgewählt. Man kann als Kohlenhydratquelle eine oder mehrere dieser Kohlenhydrate wählen.

Die gemäß diesem Aspekt offenbarten notwendigen Substanzen können in einem Nährmedium, insbesondere in einem Zellkulturmedium, verwendet werden, welches Glutamin beinhaltet, oder welches Glutamin-frei ist, oder geringe Glutaminkonzentration beinhaltet, zum Beispiel weniger als 8 mM, oder das Nährmedium, insbesondere Zellkulturmedium, kann Glutaminersatzstoffe beinhalten, zum Beispiel Alanyl-Glutamin oder Glycyl-Glutamin oder das Nährmedium, insbesondere Zellkulturmedium, kann eine Mischung mehrerer glutaminhaltiger Peptide beinhalten.

Die gemäß diesem Aspekt offenbarten notwendigen Substanzen können bevorzugt in einem Nährmedium, insbesondere in einem Zellkulturmedium, verwendet werden, welches Asparagin oder Asparaginersatzstoffe beinhaltet. Die Konzentration von Asparagin oder der Asparaginersatzstoffe kann bevorzugt zum Beispiel höher als 0,05 g/l, mehr bevorzugt höher als 0,15 g/l, besonders bevorzugt höher als 0,3 g/l, am bevorzugtesten höher als 0,6 g/l sein. Asparaginersatzstoffe können bevorzugt z.B. asparaginhaltige Peptide sein, zum Beispiel aber nicht limitiert Leu-Asn (Sigma, Katalognummer: L0641), oder Ile-Asn (Sigma, Katalognummer: I3635), oder Glu-Asn-Gly (Sigma, Katalognummer: P5148). Die Peptide können bevorzugt grundsätzlich 2 Aminosäuren oder mehr als 2 Aminosäuren, insbesondere 3 oder 4 Aminosäuren, beinhalten.

Die gemäß diesem Aspekt offenbarten notwendigen Substanzen können bevorzugt in einem Nährmedium, insbesondere in einem Zellkulturmedium, verwendet werden, welches Glutamat oder Glutamatersatzstoffe beinhaltet. Die Konzentration von Glutamat oder Glutamatersatzstoffe kann bevorzugt zum Beispiel höher als 0,05 g/l, mehr bevorzugt höher als 0,15 g/l, besonders bevorzugt höher als 0,3 g/l, am bevorzugtesten höher als 0,6 g/l sein. Glutamatersatzsatzstoffe können bevorzugt z.B. Glutamathaltige Peptide sein, zum Beispiel aber nicht limitiert Asp-Glu (Sigma, Katalognummer: A1916-250MG), Glu-Glu (Sigma, Katalognummer: G3640-25MG), Glu-His (Sigma, Katalognummer: G6882-100MG), Glu-Leu (Sigma, Katalognummer: G7007-10MG), Glu-Lys (Sigma, Katalognummer: G5136-25MG). Die Peptide können bevorzugt grundsätzlich 2 Aminosäuren oder mehr als 2 Aminosäuren, insbesondere 3 oder 4 Aminosäuren, beinhalten.

Offenbart ist auch die Bereitstellung eines Nährmediums, einer Zellkultur oder eines Verfahrens zur Verbesserung oder Verlängerung der Prozessdauer, des Zellwachstums einer Zellkultur, umfassend mindestens eine Zelle, in einem wässrigen System, bevorzugt in einem Nährmedium, insbesondere in einem Zellkulturmedium, wobei das Zellwachstum über die Zugabe der obengenannter Substanzen, bevorzugt in einem Nährmedium, insbesondere in einem Zellkulturmedium, verbessert wird. Insbesondere stellt die Erfindung ein Nährmedium, eine Zellkultur und ein Verfahren zur Erzielung höheres Zellwachstum oder zur Verlängerung der Zellvitalität oder zur Reduzierung der metabolischen Nebenproduktbildung einer Zellkultur, umfassend mindestens eine Zelle, in einem wässrigen System bereit, bevorzugt in einem Nährmedium, insbesondere in einem Zellkulturmedium, wobei das Zellwachstum über die Zugabe der organischen Säuren in einem Nährmedium, insbesondere in einem Zellkulturmedium, verbessert wird.

Gemäß der Erfindung ist das Nährmedium dadurch gekennzeichnet, dass das Nährmedium bevorzugt eine organische Säure oder Salze dieser beinhaltet. Durch die Zugabe der Substanzen wird das Zellwachstum und/oder die Produktbildung erhöht, oder die Kulturdauer verlängert und/oder die Bildung von metabolischen Nebenprodukten reduziert.

Gemäß der Offenbarung ist die Zellkultur dadurch gekennzeichnet, dass die Zellkultur in einem Medium mit erfindungsgemäßen Substanzen überleben kann oder teilen kann oder die höchste erreichte Zellkonzentration (peak cell density) erhöht wird, oder die stationäre Wachstumsphase verlängert wird oder die Absterbephase verlangsamt wird, oder die Bildung der Zell-metabolischen Nebenprodukte, zum Beispiel Ammonium oder Lactat reduziert wird, wobei das Medium bevorzugt niedrige Glutaminkonzentration oder kein Glutamin beinhalten kann, und hohe Asparaginkonzentration beinhalten kann, Glukose oder andere Kohlenhydrate beinhalten kann, insbesondere Glukosefrei ist oder Galaktose beinhaltet. Anstelle der hohen Asparaginkonzentration kann bevorzugt auch eine hohe Glutamatkonzentration beinhaltet sein.

Zellwachstum ist erfindungsgemäß das Wachstum der Zellkultur, primär, aber nicht ausschließlich hervorgerufen durch mindestens eine Zellteilung mindestens einer Zelle. Wird das Zellwachstum erhöht, so wird dies primär durch eine Erhöhung der Zellteilungsfrequenz (Zellwachstumsrate, auch µ genannt) verursacht.

Die stationäre Wachstumsphase einer Zellkultur ist eine dem Fachmann bekannte typische Wachstumsphase einer Wachstumskurve einer Zellkultur. Diese Wachstumskurve beinhaltet eine Anlaufphase, eine exponentielle Wachstumshase, eine stationäre Wachstumsphase und eine Absterbephase. Der Verlauf dieser Phasen kann variieren. Auch können Verlauf und Dauer dieser Phasen beeinflusst werden, beispielsweise durch Zugabe der erfindungsgemäßen Substanzen in einem Nährmedium, insbesondere in einem Zellkulturmedium.

Unter dem Integral der Lebendzellkonzentration wird die Lebendzellkonzentration über einen bestimmten Zeitraum hinweg gemeint (Renard, J. M., et.al., Biotechnology Letters, 1988, 10(2): 91-96). Das Integral der Lebendzellkonzentration ist also insbesondere die Anzahl aller lebenden Zellen über einen Prozesszeitraum, insbesondere über den Zeitraum der Zellkultivierung. Das Integral der Lebendzellkonzentration kann entweder durch die Anzahl der Zellen oder durch die Dauer des Prozesses verändert werden. Erfindungsgemäß bevorzugt erfolgt durch ein erfindungsgemäßes Nährmedium oder Verfahren eine Vergrößerung des Integrals der Lebendzellkonzentration.

Erfindungsgemäß bevorzugt wird das Zellwachstum verbessert oder die höchste erreichte Zellkonzentration (peak cell density) erhöht, oder die Stationäre Wachstumsphase verlängert oder die Absterbephase verlangsamt, oder die Bildung der Zell-metabolischen Nebenprodukte, zum Beispiel Ammonium oder Lactat reduziert.

Erfindungsgemäß bevorzugt handelt es sich bei dem erfindungsgemäßen Verfahren um ein Batch-Verfahren, um ein Split-batch Verfahren (wiederholter Satzbetrieb), um ein Fed-batch-Verfahren, um ein kontinuierliches Verfahren oder um ein Perfusionsverfahren.

Erfindungsgemäß bevorzugt wird in einem erfindungsgemäßen Verfahren die Zellkultur, umfassend mindestens eine Zelle, zur Herstellung eines biologischen Produktes verwendet. Erfindungsgemäß bevorzugt ist das biologische Produkt ausgewählt aus mindestens einem Polypeptid, mindestens einem Protein, mindestens einem Virusbestandteil, mindestens einem Virus oder aus einer Mischung aus diesen. Erfindungsgemäß bevorzugt ist das biologische Produkt mindestens ein Impfstoff.

Für die Produktion eines gewünschten biotechnologischen Produkts, insbesondere eines Polypeptids, Proteins oder eines Impfstoffs, werden je nach Produkt entweder prokaryotische Zellen, zum Beispiel Bakterien, oder eukaryontische Zellen, zum Beispiel Hefezellen oder Pflanzenzellen, insbesondere aber tierische Zellen, bevorzugt Säugerzellen, eingesetzt. Das Gen des zu produzierenden Produkts kann in den Wirtszellen in natürlicher Weise oder rekombinant vorkommen. Das Produktspektrum kann variieren von Peptiden mit wenigen Aminosäuren bis zu ganzen Viren. Besonders bei komplexen Proteinen, die nach der Translation in einem post-translationalen Prozess verändert, insbesondere glykosyliert werden, werden bevorzugt tierische Zellen eingesetzt. In einer weiteren Variante ist vorgesehen, andere eukaryontische Zellen einzusetzen, beispielsweise Hefe.

Erfindungsgemäß bevorzugt wird das biologische Produkt aus Zellkultur und/oder aus dem Zellkulturüberstand, oder aus den Zellen (intrazellulär) selbst gewonnen.

Erfindungsgemäß bevorzugt kann das biologische Produkt auch die Zelle selbst sein, zum Beispiel es kann eine Primärzelle, eine Stammzelle, ein Primärzellverband, ein Gewebebestandteil, oder ein komplettes Gewebe sein.

Erfindungsgemäß bevorzugt enthält die mindestens eine Zelle mindestens ein Gen, das das biologische Produkt kodiert. Das Gen kann ein Fremdgen sein, also ein Gen, welches in natürlicher Art und Weise in der Produktionszelle nicht vorkommt oder das Gen kann ein zelleigenes Gen sein, welches in natürlicher Art und Weise in der Produktionszelle vorkommt. So ein zelleigenes Gen kann zum Beispiel in der Produktionszelle aktiv oder inaktiv vorkommen. Wenn das zelleigene Gen inaktiv war, kann das Gen durch externer Zugriff aktiviert worden sein.

Erfindungsgemäß ist auch ein Verfahren zur Kultivierung mindestens einer Zelle als Zellkultur, wobei die mindestens eine Zelle in einem Nährmedium, insbesondere in einem Zellkulturmedium, kultiviert und dabei erfindungsgemäß bevorzugt in ihrem Zellwachstum gemäß einem erfindungsgemäßen Verfahrens stimuliert wird.

Erfindungsgemäß ist auch ein Verfahren zur Gewinnung eines biologischen Produkts aus mindestens einer Zelle, wobei die mindestens eine Zelle gemäß einem erfindungsgemäßen Verfahren kultiviert und daraus das biologische Produkt gewonnen wird.

Es wurde überraschender Weise gefunden, dass gerade diese organische Säuren (Citronensäure, Bernsteinsäure, Äpfelsäure, α-Keto-Glutarsäure, Fumarsäure, Oxalessigsäure, Isocitronensäure, Oxalbernsteinsäure, Weinsäure, Adipinsäure, Milchsäure) Zellwachstum und/oder Polypeptidproduktion fördern und/oder Stoffwechsel-Nebenproduktbildung unterdrücken, besonders, wenn man höhere Konzentrationen einsetzt. Insbesondere, wenn man diese Komponenten in einem glutaminfreien oder glutaminarmen Nährmedium einsetzt, oder in einem Medium mit hoher Asparaginkonzentration, oder Asparaginersatzstoffkonzentration einsetzt, oder in einem Medium mit hoher Glutamatkonzentration, oder mit hoher Glutaminersatzstoffkonzentration einsetzt, oder in einem Medium mit oder ohne Glukose einsetzt, oder mit Galaktose einsetzt. In dieser Hinsicht unterscheidet sich die vorliegende Erfindung von der Lehre nach der EP 0 435 911 B1. In der genannten Patentschrift wird vorgeschlagen, höhere Glutaminkonzentration (größer als 8 mM) in Medien einzusetzen, während die vorliegende Erfindung bevorzugt eine reduzierte Glutaminkonzentration, insbesondere ein glutaminfreies Nährmedium, verwendet.

In der EP 0 435 911 B1, (Seite 8, Zeile 14, und Seite 14) wurden die Komponenten Succinat, Malat, α-Keto-Glutarat, Fumarat und Citrat als Chelatoren für Metalionen in einem Nährmedium, insbesondere in einem Zellkulturmedium, getestet. Anhand der durchgeführten Experimente wurde herausgefunden, dass Citrat ein guter Chelator für die Metallionen ist (Seite 22, Anspruch 5). Außer Citrat scheinen die anderen getesteten organischen Säuren kein zufrieden stellendes Resultat geliefert zu haben. Da das Ziel war, in EP 0 435 911 B1 einen guten Metallchelator zu finden, hat man sehr geringe Konzentrationen der getesteten organischen Säuren eingesetzt (Malat = 10,7 mg/l, α-Keto-Glutarat = 5,9 mg/l, Succinat = 0,96 mg/l, Fumarat = 0,88 mg/l). Außerdem werden in EP 0 435 911 B1 diese Substanzen ausdrücklich in einem Medium mit einer Glutaminkonzentration von größer als 8 mM eingesetzt, weil dies die Erfindung dieser Druckschrift ist. Ein anderer Unterschied zwischen der Lehre der EP 0 435 911 B1 und der vorliegenden Erfindung ist, dass man die offenbarten Substanzen als Modulatoren des Stoffwechsels einsetzt. In diesem Zusammenhang können die Substanzen in einem Nährmedium, insbesondere in einem Zellkulturmedium, bevorzugt verwendet werden, welches Kohlenhydrate und/oder Glutamin beinhaltet. Die organischen Säuren können aber auch bevorzugt in einem Nährmedium, insbesondere in einem Zellkulturmedium, verwendet werden, welches keine Kohlenhydrate und/oder kein Glutamin enthält. In EP 0 435 911 B1 sind die Hauptenergie- und Kohlenstoffquellen der Zellen Glucose und Glutamin, sodass die Substanzen in der geringen Konzentration keine Bedeutung als Stoffwechsel-Modulator haben.

Es wurde überraschender Weise gefunden, dass gerade einer der bekanntesten Stoffwechsel-Nebenprodukte von Zellen, nämlich Lactat, das Zellwachstum und das Integral der Lebendzellkonzentration fördert. Es gibt zahlreiche Publikationen darüber, dass Lactat ein Stoffwechselnebenprodukt ist und sogar für die Zellen toxisch sei. Es wurde überraschender Weise herausgefunden, dass Lactat einen positiven Effekt auf das Zellwachstum hat, wenn das Nährmedium, insbesondere das Zellkulturmedium, kein Glutamin enthält oder geringe Konzentration von Glutamin enthält, zum Beispiel eine Glutaminkonzentration von weniger als 8 mM enthält, oder wenn Glutamin mit glutaminhaltigen Glutaminersatzstoffen ersetzt worden ist, oder wenn das Medium Asparagin enthält, besonders wenn Asparagin in höherer Konzentration vorkommt, z.B. die Asparaginkonzentration höher als 0,3 g/l ist, oder wenn Asparagin mit asparaginhaltigen Ersatzstoffen ersetzt worden ist, oder wenn das Medium Glutamat enthält, besonders wenn die Glutamatkonzentration höher als 0,3 g/l ist, oder wenn Glutamat mit glutamathaltigen Ersatzstoffen ersetzt worden ist, oder das Medium mit oder ohne Glukose formuliert worden ist, besonders wenn das Medium glukosefrei ist, insbesondere wenn das Medium Galactose enthält. Der positive Effekt von Lactat ist besonders dann ersichtlich, wenn das Medium kein Glutamin beinhaltet und als Kohlenstoffquelle andere Kohlenhydrate beinhaltet als Glukose, besonders wenn das Medium Galactose beinhaltet.

Ein bevorzugter Aspekt der Offenbarung betrifft somit ein Nährmedium, insbesondere ein Zellkuturmedium, zur Kultivierung einer Zelle, oder zur Gewinnung eines Polypeptids, eines Proteins oder eines Impfstoffes (zum Beispiel eines Virus) aus Organismen, insbesondere aus Zellen oder Zellkulturen, bevorzugt aus eukaryontischen Zellen oder Zellkulturen, wobei die Organismen in einem geeigneten Medium kultiviert werden und das gewünschte Polypeptid oder Protein oder der Impfstoff aus den Organismen und/oder dem Kulturüberstand gewonnen wird, dadurch gekennzeichnet, dass das Zellwachstum durch die Zugabe von Citronensäure, Bernsteinsäure, Äpfelsäure, α-Keto-Glutarsäure, Fumarsäure, Oxalessigsäure, Isocitronensäure, Oxalbernsteinsäure, Weinsäure, Adipinsäure, Milchsäure und Mischungen davon sowie Salze oder Komplexe dieser Säuren in einem Nährmedium, insbesondere in einem Zellkulturmedium, stimuliert wird.

Ein zweiter bevorzugter Aspekt der Offenbarung betrifft ein Verfahren zur Kultivierung einer Zelle, oder zur Gewinnung eines Polypeptids, eines Proteins oder eines Impfstoffes (zum Beispiel eines Virus) aus Organismen, insbesondere aus Zellen oder Zellkulturen, bevorzugt aus eukaryontischen Zellen oder Zellkulturen, wobei die Organismen in einem geeigneten Medium kultiviert werden und das gewünschte Polypeptid oder Protein oder der Impfstoff aus den Organismen und/oder dem Kulturüberstand gewonnen wird, wobei das Verfahren dadurch gekennzeichnet ist, dass die Organismen mit einem Nährmedium kontaktiert werden, welches mindestens eine der folgenden Substanzen enthält: Citronensäure, Bernsteinsäure (erfindungsgemäß), Apfelsäure, α-Keto-Glutarsäure, Fumarsäure, Oxalessigsäure, Isocitronensäure, Oxalbernsteinsäure, Weinsäure, Adipinsäure, Milchsäure und Mischungen davon sowie Salze oder Komplexe dieser Säuren.

Die gemäß diesem Aspekt der Offenbarung besonders bevorzugte Wachstumsstimulierung durch Zugabe der obengenanten Substanzen ins Nährmedium wird dadurch erzielt, dass das Nährmedium Glutamin-arm ist, zum Beispiel eine Glutaminkonzentration von kleiner als 8 mM, bevorzugt kleiner als 5 mM, besonders bevorzugt kleiner als 3 mM, am meisten bevorzugt kleiner als 2 mM aufweist.

Die gemäß diesem Aspekt der Offenbarung besonders bevorzugte Wachstumsstimulierung durch Zugabe der obengenanten Substanzen ins Nährmedium, insbesondere in das Zellkulturmedium, wird dadurch erzielt, dass das Nährmedium und/oder Feedmedium frei von Glutamin ist.

Die gemäß diesem Aspekt der Offenbarung besonders bevorzugte Wachstumsstimulierung durch Zugabe der obengenanten Substanzen ins Nährmedium wird dadurch erzielt, dass das Nährmedium glutaminhaltige Glutamin-Ersatzstoffe beinhaltet. Die Glutaminhaltige Glutaminersatzstoff-Konzentration in Nährmedium ist dabei bevorzugt mindestens 50 mg/l, bevorzugter mindestens 250 mg/l, besonders bevorzugt mindestens 500 mg/l, am meisten bevorzugt mindestens 1000 mg/l. Die Glutaminhaltige Glutaminersatzstoff-Konzentration in Nährmedium ist dabei bevorzugt auch mindestens 0,05 g/l, bevorzugter mindestens 0,2 g/l, besonders bevorzugt mindestens 0,8 g/l, am meisten bevorzugt mindestens 3,2 g/l.

Die gemäß diesem Aspekt der Offenbarung besonders bevorzugte Wachstumsstimulierung durch Zugabe der obengenanten Substanzen ins Nährmedium wird dadurch erzielt, dass das Nährmedium Asparagin oder Asparagin-Ersatzstoffe beinhaltet. Die Asparagin- oder Asparagin-Ersatzstoffkonzentration im Nährmedium ist bevorzugt mindestens 0,05 g/l, bevorzugter mindestens 0,15 g/l, besonders bevorzugt mindestens 0,3 g/l, am meisten bevorzugt mindestens 0,6 g/l. Die gemäß diesem Aspekt der Offenbarung besonders bevorzugte Wachstumsstimulierung durch Zugabe der obengenanten Substanzen ins Nährmedium wird dadurch erzielt, dass das Nährmedium Glutamat oder Glutamat-Ersatzstoffe beinhaltet. Die Glutamat- oder Glutamat-Ersatzstoffkonzentration im Nährmedium ist bevorzugt mindestens 0,05 g/l, bevorzugter mindestens 0,15 g/l, besonders bevorzugt mindestens 0,3 g/l, am meisten bevorzugt mindestens 0,6 g/l.

Die gemäß diesem Aspekt der Offenbarung besonders bevorzugte Wachstumsstimulierung durch Zugabe der obengenanten Substanzen ins Nährmedium wird dadurch erzielt, dass das Nährmedium Glucose-frei ist.

Die gemäß diesem Aspekt der Offenbarung besonders bevorzugte Wachstumsstimulierung durch Zugabe der obengenanten Substanzen ins Nährmedium wird dadurch erzielt, dass das Nährmedium Galactose enthält. Die Galactosekonzentration ist mindestens 0,1 g/l, bevorzugt mindestens 1 g/l, besonders bevorzugt mindestens 5 g/l, am meisten bevorzugt mindestens 25 g/l. Die gemäß diesem Aspekt der Offenbarung besonders bevorzugte Wachstumsstimulierung durch Zugabe der obengenanten Substanzen ins Nährmedium wird dadurch erzielt, dass das Nährmedium Lactat enthält. Die Lactatkonzentration ist mindestens 0,1 g/l bevorzugt mindestens 1 g/l, besonders bevorzugt mindestens 5 g/l, am meisten bevorzugt mindestes 25 g/l.

Die gemäß diesem Aspekt der Offenbarung besonders bevorzugte Wachstumsstimulierung durch Zugabe der obengenanten Substanzen ins Nährmedium wird dadurch erzielt, dass das Nährmedium und/oder Feedmedium frei von Glutamin oder glutaminhaltigen Ersatzstoffen ist.

Die gemäß diesem Aspekt der Offenbarung besonders bevorzugte Wachstumsstimulierung durch Zugabe der obengenanten Substanzen ins Nährmedium, insbesondere ins Zellkulturmedium, wird dadurch erzielt, dass das Feedmedium glutaminhaltige Glutamin-Ersatzstoffe beinhaltet. Die glutaminhaltige Glutaminersatzstoff-Konzentration im Feedmedium ist bevorzugt mindestens 0,2 g/l, bevorzugter mindestens 1 g/l, besonders bevorzugt mindestens 5 g/l, am meisten bevorzugt mindestens 10 g/l.

Die gemäß diesem Aspekt der Offenbarung besonders bevorzugte Wachstumsstimulierung durch Zugabe der obengenanten Substanzen ins Nährmedium, insbesondere ins Zellkulturmedium, wird dadurch erzielt, dass das Feedmedium Asparagin oder Asparagin-Ersatzstoffe beinhaltet. Die Asparagin- oder Asparagin-Ersatzstoffkonzentration im Feedmedium ist bevorzugt mindestens 0,2 g/l, bevorzugt mindestens 1 g/l, besonders bevorzugt mindestens 5 g/l, am meisten bevorzugt mindestens 10 g/l.

Die gemäß diesem Aspekt der Offenbarung besonders bevorzugte Wachstumsstimulierung durch Zugabe der obengenannten Substanzen ins Nährmedium, insbesondere ins Zellkulturmedium, wird dadurch erzielt, dass das Feedmedium Glutamat oder Glutamat-Ersatzstoffe beinhaltet. Die Glutamat- oder Glutamat-Ersatzstoffkonzentration im Feedmedium ist bevorzugt mindestens 0,2 g/l, bevorzugter mindestens 1 g/l, besonders bevorzugt mindestens 5 g/l, am meisten bevorzugt mindestens 10 g/l.

Die gemäß diesem Aspekt der Offenbarung besonders bevorzugte Wachstumsstimulierung durch Zugabe der obengenanten Substanzen ins Nährmedium, insbesondere ins Zellkulturmedium, wird dadurch erzielt, dass das Feedmedium Lactat oder Lactat-Derivate beinhaltet. Die Lactat. oder Lactat-Derivatkonzentration im Feedmedium ist bevorzugt mindestens 1 g/l, bevorzugter mindestens 5 g/l, besonders bevorzugt mindestens 25 g/l, am meisten bevorzugt mindestens 100 g/l.

Die gemäß diesem Aspekt der Offenbarung besonders bevorzugte Wachstumsstimulierung durch Zugabe der obengenanten Substanzen ins Nährmedium, insbesondere in das Zellkulturmedium, wird dadurch erzielt, dass das Feedmedium Glucose-frei ist. Die Glucosekonzentration im Feedmedium ist bevorzugt maximal 100 g/l, bevorzugt maximal 25 g/l, besonders bevorzugt maximal 5 g/l, am meisten bevorzugt maximal 1 g/l.

Die gemäß diesem Aspekt der Offenbarung besonders bevorzugte Wachstumsstimulierung durch Zugabe der obengenanten Substanzen ins Nährmedium, insbesondere in das Zellkulturmedium, wird dadurch erzielt, dass das Feedmedium Galactose beinhaltet. Die Galactosekonzentration im Feedmedium ist bevorzugt mindestens 1 g/l, bevorzugt mindestens 5 g/l, besonders bevorzugt mindestens 25 g/l, am meisten bevorzugt mindestens 100 g/l.

Offenbart ist auch eine Zelle oder eine Zellkultur, die dadurch gekennzeichnet ist, dass die Zellkultur in einem Medium mit hier offenbarten Substanzen überleben kann oder teilen kann oder deren höchste erreichte Zellkonzentration (peak cell density) erhöht ist, oder deren stationäre Wachstumsphase verlängert ist oder deren Absterbephase verlangsamt ist, oder deren Bildung der Zell-metabolischen Nebenprodukte, zum Beispiel Ammonium oder Lactat reduziert ist.

Aufgrund der Wachstumsstimulierung durch die Zugabe der offenbarten Substanzen ins Nährmedium, insbesondere in das Zellkulturmedium, kann bevorzugt bei einer Hochzelldichtefermentation (höchste erreichte Gesamtzellkonzentration während der Fermentation beispielsweise insbesondere >1x10⁵ Zellen/ml, vorzugsweise > 1x10⁶ Zellen/ml im Großmaßstab (Arbeitsvolumen > 1 I, zum Beispiel 30 - 20 000 I) die höchste erreichte Zellkonzentration (peak cell density) erhöht werden, oder die stationäre Wachstumsphase verlängert werden, wobei die Zellvitalität länger hoch bleibt, d.h. das Integral der Lebendzellkonzentration erhöht wird und dadurch die Produktausbeute erhöht wird.

Das erfindungsgemäße Verfahren ist grundsätzlich zur Herstellung beliebiger Polypeptide, Proteine oder Impfstoffe, wie zum Beispiel zur Virusproduktion, geeignet. Bevorzugt sind jedoch Polypeptide, die glykosyliert oder nicht glykosyliert sein können. Die Polypeptide können beispielsweise natürliche, humane Polypeptide oder rekombinante Varianten humaner Polypeptide sein.

Das offenbarten Nährmedium ist bevorzugt zur Herstellung der Polypeptide mit post-translationalen Modifikationen, insbesondere zur Herstellung von Glykoproteinen geeignet. Die Zugabe der offenbarten Substanzen in ein Nährmedium, insbesondere in ein Zellkulturmedium, kann bestimmte Glykosylierungspathways fördern oder inhibieren und somit für die Bildung einer gewünschten Glykostruktur des gewünschten Polypeptides oder des Impfstoffes förderlich sein. Die erfindungsgemäßen Substanzen können einzeln oder in Kombination miteinander bestimmte post-translationale Modifikation in der Zelle fördern oder inhibieren, mit dem Resultat, dass in der Produktion die gewünschten Polypeptidvarianten oder Impfstoffvarianten exprimiert werden. Beispielsweise können die erfindungsgemäßen Substanzen die Proteinsialilierung oder die Proteingalactosilierung erhöhen.

Durch die Verwendung der offenbarten Substanzen und Nährmedien läuft der pH-Wert des Zellkultivierungsprozesses sehr atypisch am oberen Ende des Sollwertes. Es ist bei Zellkultivierungsprozessen üblich, dass der pH-Wert des Prozesses mit pH 7,0 bis pH 7,1 startet, und dass die Zellen durch ihre Stoffwechselaktivitäten Säuren, insbesondere Lactat in die Kultur ausscheiden. Dadurch verschiebt sich der pH-Wert der Kultur normalerweise in Richtung saurer Bereich. Es wurde überraschender Weise festgestellt, dass sich der pH-Wert bei Anwendung des erfindungsgemäßen Zellkultivierungsprozesses wegen des veränderten Metabolismus der Zellen in Richtung basische pH-Werte verschiebt. Dies könnte damit zusammenhängen, ohne an die Theorie gebunden zu sein, dass die Zellen einen besseren Metabolismus haben und keine Säuren mehr in die Kultur ausscheiden. Umgekehrt, sie metabolisieren Säuren. Das ist ein anderer Beweis dafür, dass die Zellen durch die erfindungsgemäßen Substanzen und Verfahren einen anderen Metabolismus haben. Als Konsequenz liegt der pH-Wert der Kultur bei pH-Werten oberhalb von 7,2 (höher als pH 7,2 oder anders formuliert basischer als pH 7,2). Dies ist für die Proteinglykosylierung, zum Beispiel aber nicht limitiert für die Proteingalactosilierung oder für die Proteinsialilierung nützlich. Einige intrazelluläre Glykosylierungsenzyme wiederum sind bei pH-Werten basischer als 7,2 aktiver, so dass sie die zu produzierenden Proteine höher glykosylieren. Das ist eine wichtige Prozesseigenschaft, die durch den Metabolismus der offenbarten Substanzen hervorgerufen wird.

Offenbart ist auch ein Kultivierungsverfahren unter Nutzung vorgenannter Kulturmedien, bei dem der pH-Wert des Zellkultivierungsprozesses vorzugsweise basischer ist als pH 7,2, insbesondere basischer als pH 7,3, bevorzugter basischer als pH 7,4, noch bevorzugter basischer als pH 7,5 sehr bevorzugt basischer als pH 7,6, am meisten bevorzugt basischer als pH 7,7.

Das offenbarte Verfahren ist grundsätzlich zur Kultivierung prokaryontischer Zellen, zum Beispiel Bakterien, oder eukaryontischer Zellen, beispielsweise Hefen oder tierische Zellen, geeignet. Der Begriff "tierische Zelle" umfasst nicht-Säugerzellen und Säugerzellen. Beispiele für nicht-Säugerzellen sind Zellen von Spodoptera frugiperda, Aedes aegypti, Aedes albopictus. Erfindungsgemäße Organismen für diese Erfindung sind jedoch Säugerzellen. Die Säugerzellen können Primärzellen sein, erfindungsgemäß sind jedoch kontinuierliche Zelllinien. Die geeigneten kontinuierlichen Zelllinien können beispielsweise einen humanen Ursprung haben, zum Beispiel PER-C6, human liver cells (Hep G2, HB 8065), human lung cells (W138, ATCC CCL 75), human cervical carcinoma cells (HeLa, ATCC CCL 2). Die kontinuierlichen Zelllinien können aber auch einen tierischen Ursprung haben, beispielsweise aus Maus oder Hamster abgeleitet sein, zum Beispiel SV-40 immortalisierte monkey kidney cells (COS-7, ATCC CRL 1651), canine kidney cells (MDCK), monkey kidney cells (CV1, ATCC CCL 70), afrikan green monkey kidney cells (VERO-76, ATCC CRL-1587), baby hamster kidney cells (BHK, ATCC CCL 10), chine-se hamster ovary cells (CHO-DG44, Urlaub und Chasin, Proc. Natl. Acad. Sci. USA, 1980, 77: 4216, oder CHO-DUKX, oder CHO-K1 mit ATCC-Nummer von ATCC CCL 61), lymphocytic cells (Jurkat T-cell line), buffalo rat liver cells (BRL 3A, ATCC CRL 1442), mouse mammary tumor cells (MMT 060562, ATCC CCL 51), SP2/0 Zellen, Myelomazellen (zum Beispiel NS0), Hybridomazellen und Triomazellen. Die Hybrid-Zelllinien können von irgendeiner Spezies, umfassend Mensch und Maus, stammen. Die Abkömmlinge der obengenannter Zellen sind ebenfalls geeignet in Kultivierung in offenbarten Nährmedien und erfindungsgemäßen Verfahren.

Die gemäß dieser Erfindung bevorzugten Zellen sind allerdings aus einer Säugerzelllinie und sind keine Hybridomazellen. Die gemäß der Erfindung besonders bevorzugten Zelllinien sind alle Varianten der CHO, NS0, PERC-6 und BHK-21-Zelllinien, wobei die Zelllinie mit einer exogenen Nukleinsäure transformiert sind, wobei die exogene Nukleinsäure für das interessierende Polypeptid kodiert. "Exogene Nukleinsäure" bedeutet eine Nukleinsäuresequenz, welche in der Wirtszelle nicht vorkommt. Die exogene Nukleinsäure kann auch homolog zu einer Nukleinsäure der Wirtszelle sein, die aber in der Wirtszelle in dieser Position normaler Weise nicht zu finden ist.

Die gemäß dieser Erfindung bevorzugten Zellen sind in der Lage, in den Medien mit hier offenbarten Substanzen und Verfahren überleben zu können oder teilen zu können oder deren höchste erreichte Zellkonzentration (peak cell density) erhöht ist, oder deren stationäre Wachstumsphase verlängert ist oder deren Absterbephase verlangsamt ist, oder deren Bildung der zell-metabolischen Nebenprodukte, zum Beispiel Ammonium oder Lactat reduziert ist. Der Metabolismus der Zellen kann in Medien mit hier offenbarten Substanzen oder in erfindungsgemäßen Verfahren angepasst sein. Diese Anpassung kann durch gezielte molekular biologische Veränderung der Zellen oder durch die Adaptation der Zellen in den erfindungsgemäßen Medien und Verfahren geschehen.

Der Begriff "Klon" bedeutet, eine Abkömmling einer Mutterzelle, zum Beispiel eine der oben gezählten Zelllinien. Ein Klon kann entstehen, wenn eine Zelle andere zelluläre Eigenschaften besitzt und sich von der ursprünglichen Mutterzelle dadurch unterscheidet. Üblicher Weise können Klone nach einem Gentransfer der Mutterzelle, zum Beispiel durch Transfektion entstehen.

Der Begriff "metabolische Optimierung" bedeutet, Anpassung oder Veränderung des Zellmetabolismus an die gewünschten Bedingungen. Die Anpassung oder Veränderung des Zellmetabolismus kann erzielt werden durch gezielte genetischer Eingriff in die Wirtszelle, in dem man bestimmte Gene in die Zelle kloniert, oder bestimmte Gene ausschaltet. Oder die Anpassung oder Veränderung des Zellmetabolismus kann erreicht werden, wenn die Zellen unter den gewünschten Verfahrensbedingungen und/oder in Nährmedien kultiviert werden, so dass die Zelle selbst sich auf die veränderten Bedingungen anpasst. Durch die Kultivierung unter den neuen Bedingungen können neuartige Zellen entstehen, dich sich selektieren und isolieren lassen, die genetisch modifiziert sind. Die genetische Modifikation zeigt sich beispielsweise durch eine Veränderung, insbesondere Verbesserung des Zellmetabolismus. Insbesondere solche Zellen mit einem solchen veränderten Zellmetabolismus wachsen im vergleich zu herkömmlichen Zellen besonders gut unter den gewünschten Bedingungen, da deren Metabolismus optimiert worden ist.

Ein Produkt gemäß der Erfindung ist ein Polypeptid ein Protein oder ein Virus, welches in den Zellen exprimiert und aus dem Kultivierungssystem, also den Zellen und/oder dem Zellmedium, geerntet wird. Es kann ein jegliches interessierendes Protein sein. Es können zum Beispiel diagnostische oder therapeutische Proteine sein, wie Interleukine, Enzyme, multimere Proteine oder Subeinheiten der multimeren Proteine, sowie Antikörper oder Antikörperfragmente. Das rekombinante Gen für das interessierende Protein kann ein Signalsequenz beinhalten, welche für die Ausscheidung des interessierenden Proteins aus der Wirtszelle verantwortlich ist. Das Protein kann aus einem transgenen Promoter oder dessen natürlich aktiven Gen-Lokus, sowie einem Immunglobolin-Genlokus in Hybridomazellen exprimiert werden. Das Protein kann auch aus einem Wirtseigenen Promoter exprimiert werden, zum Beispiel wenn eine CHO-Zelle für Proteinproduktion benutzt wird, kann das Fremdgen aus einem CHO-Promoter exprimiert werden. Das Produkt kann aber auch ein Impfstoff sein, zum Beispiel ein Virus oder virusähnliches Partikel, welches in der Lage ist mit Hilfe einer Wirtszelle sich zu replizieren. Die zu kultivierenden Zellen können adhärent oder auf Microcarriern wachsen, bevorzugt sind jedoch in Suspension wachsende Zellen. Weiterhin können die Zellen in serumhaltigen Kulturmedien (zum Beispiel in fetal bovine Serum, FBS, auch FCS genannten Serum) wachsen, bevorzugt ist aber ein Medium, welches kein Serum beinhaltet und "serum frei" genannt wird. Zellen in Serum-freien Medien können Insulin und Transferrin für optimales Wachstum benötigen. Transferrin kann zumindest partial durch Alternativen ersetzt werden, zum Beispiel durch Eisencitrat. Die meisten Zelllinien benötigen einen oder mehrere Wachstumsfaktoren, umfassend die rekombinanten Polypetide oder Proteine als Wachstumsfaktor. Das Medium kann Polypeptide beinhalten, bevorzugt ist aber ein polypeptidarmes oder ein polypeptidfreies oder ein Proteinfreies Medium. Wenn das Medium ein Polypeptid beinhaltet, ist ein rekombinant hergestelltes Polypeptid bevorzugt. Das Medium kann Peptone beinhalten, die aus tierischem Ursprung stammen können, bevorzugt sind aber Peptone, die Hefe als Ursprung haben, besonders bevorzugt sind die Peptone, die aus Pflanzen stammen. Vorzugsweise werden Kulturmedien verwendet, die peptonfrei und voll chemisch definiert sind.

Unter "Nährmedium", dem Fachmann auch als "Kulturmedium" oder "Zellkulturmedium" bekannt, wird ein Medium verstanden, das der Aufbewahrung und/oder Kultivierung von Zellkulturen beziehungsweise von mindestens einer Zelle dient. Erfindungsgemäß bevorzugt ist ein Nährmedium ein flüssiges Nährmedium, auch Kulturflüssigkeit genannt. Als Überbegriff aller Medien wird hier der Begriff "Nährmedium" eingeführt. Nährmedium umfasst zum Beispiel, aber nicht limitiert die Begriffe "Basalmedium" (Kulturmedium), "Zufütterungsmedium" (Feedmedium) und "Perfusionsmedium". Nährmedium ist also eine Flüssigkeit, welche Nährstoffe für Zellen beinhaltet. Die erfindungsgemäßen Substanzen können in einem Nährmedium mit anderen zellulären Nährstoffen gemeinsam vorkommen. Es ist aber möglich, dass die hier offenbarten Substanzen in einem Nährmedium nicht vorkommen, stattdessen aus einer anderen Lösung, zum Beispiel aus einer Stammlösung in ein Nährmedium mit oder ohne Zellen extern in die Kultur zugegeben werden. Dadurch kommen die hier offenbarten Substanzen nicht direkt über ein Nährmedium, insbesondere über ein Zellkulturmedium, sondern über einen Umweg mit den Zellen in Kontakt. Insofern ein Verfahren zur Kontaktierung der Zellen mit hier offenbarten Substanzen ist ebenfalls Teil der Erfindung. Mit "Nährmedium", Kulturmedium" oder "Basalmedium" ist ein Medium, insbesondere eine Kulturflüssigkeit gemeint, welches Zellwachstum und/oder Zellvitalität und/oder Produktbildung fördert. Bevorzugt ist das Kulturmedium flüssig.

Erfindungsgemäß bevorzugt wird ein Hochzelldichte-ZellkulturMedium eingesetzt, durch welches, eine Population von tierischen Zellen in einer bestimmten Periode der Kultivierung mit einer Lebendzellkonzentration von mindestens 1x10⁵/ml, bevorzugt mindestens 1x10⁶/ml erreicht werden kann, mit notwendigen Nährstoffen zu versorgen. Kulturmedien, die solche Hochzelldichten mit Nährstoffen versorgen, werden mit folgenden beispielhaften Substanzen aufgestockt: Mindestens eine Aminosäure, üblicher Weise mehrere Aminosäuren bis zu alle Aminosäuren inklusive Cystin. Mindestens eine Energiequelle, üblicherweise eine Kohlenhydratquelle, beispielsweise Glucose, anorganische Salze, Vitamine, beispielsweise Vitamin C und/oder Vitamin E. Spurenelemente, auch definiert als anorganische Komponente, die in mikromolaren Konzentrationen vorkommen. Puffer, bis zu vier Nukleoside oder Nukleotide, Antioxidanzien sowie Gluthation. Lipide, beispielsweise Cholesterol, Phosphatidylcholin oder Lipidvorstufen beispielsweise Cholin oder Inositol. Ein Hochzelldichte-Medium wird bevorzugt mit mindestens einem der oben aufgezählten Komponenten versetzt und kann die oben aufgezählten Komponenten in hohen Konzentrationen beinhalten.

Das Nährmedium, insbesondere das Zellkulturmedium, kann optional mit einem oder mehreren oder allen Komponenten der folgenden Kategorien supplementiert werden:
a) Hormone und andere Wachstumsfaktoren, zum Beispiel IGF-1, Insulin, Transferrin, epidermaler Wachstumsfaktor
b) Salze und Puffer, zum Beispiel Kalzium, Magnesium, und Phosphate
c) Nukleoside und Basen, zum Beispiel Adenosin, Thymidin, Hypoxanthin
d) Protein- und Gewebehydrolysate.

Die Formulierung der Kulturmedien, denen erfindungsgemäße Substanzen zugesetzt werden können, kann bevorzugt einer der publizierten Formulierungen entsprechen. Die Medienformulierungen können bevorzugt in der publizierten Art und Weise eingesetzt werden, oft werden sie aber modifiziert und an die Ansprüche der verwendeten Zelle angepasst. Beispiele für geeignete publizierte Kulturmedienformulierungen sind, Roswell Park Memorial Institute (RPMI) 1640 Medium, L-15-Medium, Dulbecco's Modified Eagle's Medium (DMEM), Eagle's Minimal Essentail Medium (MEM), Ham's F12 Medium, oder Iscoves' Modified DMEM. Diese publizierten Medienformulierungen können allein oder in Mischung in einem beliebigen Mischungsverhältnis als Basalmedienpulver verwendet werden. Die Formulierung der Kulturmedien, denen die erfindungsgemäße Substanzen zugesetzt werden können, kann besonders bevorzugt ein kommerzielles Nährmedium sein, dessen Rezeptur nicht publiziert sind, beispielsweise aber nicht limitiert, CD-CHO Medium von GIBCO, Ex-Cell 325 PF CHO Medium von Sigma (Cat. Nr.: 14340C).

"Feedmedium" oder "Zufütterungsmedium" ist ein Medium, welches während der Zellkultivierung in das Kultivierungssystem zugegeben wird, d.h. Feedmedien werden in Kultivierungssystemen gegeben, nach dem die Zellen mit einem anderen Medium, zum Beispiel einem Kulturmedium (Basalmedium) in Kontakt gekommen sind. Feedmedien beinhalten Substanzen, die das Zellwachstum und/oder die Zellvitalität und/oder die Produktbildung fördern. Durch Zugabe der Feedmedien kann die Prozesszeit verlängert werden, weil die Zellvitalität länger hoch bleiben kann. Die Feedmedien können eine oder mehrere Energiequellen beinhalten, beispielsweise Kohlenhydrate, zum Beispiel Glucose. Glucose kann im Feedmedium enthalten sein oder getrennt aus einer anderen Stammlösung in das Kultivierungssystem zugefüttert werden. Das Feedmedium kann eine oder mehrere Aminosäuren beinhalten. Feedmedien können nur essentielle Aminosäuren oder nur nicht-essentielle Aminosäuren, oder auch eine Mischung der beiden Aminosäuregruppen beinhalten. Das Feedmedium kann Glutamin beinhalten, oder Glutaminfrei sein oder Glutaminersatzstoffe beinhalten ,oder glutaminhaltige Glutaminersatzstoffe beinhalten oder eine Mischung aus Glutamin und glutaminhaltigen Glutaminersatzstoffe beinhalten. Glutamin kann also in dem Feedmedium mit anderen Substanzen kombiniert sein, oder getrennt aus einer Stammlösung als Einzelsubstanz in das Kultivierungssystem zugefüttert werden. Feedmedien können nur einfache Konzentrate des Basalmediums sein, bevorzugt sind aber Feedmedien, die mehr als einfach konzentriert sind, zum Beispiel zweifach oder höher konzentrierte Formen des Basalmediums. Feedmedienrezepturen ähneln Basalmedienrezepturen, d.h. in der Regel beinhalten die Feedmedien dieselben oder ein Teil der Substanzen, die in Basalmedien vorkommen. Feedmedien können sich von Basalmedien in ihren anorganischen Komponenten unterscheiden. Sie können zum Beispiel ein Basalmedium ohne Salze, oder eine salzarme Variante des Basalmediums sein. Feedmedien können aber auch Substanzen umfassen, die in Basalmedienrezepturen nicht vorkommen.

Als "glutaminhaltige Substanzen", "Glutamin-Derivate" oder "glutaminhaltige Glutaminersatzstoffe" werden Substanzen gemeint, die D- oder L-Glutamin, insbesondere L-Glutamin in einem Molekül beinhalten. Dieses Molekül kann das D- oder L-Glutamin in verschiedenster Art und Weise beinhalten, beispielsweise aber nicht limitiert kann Glutamin mit einem anderen Substanz kovalent gebunden sein. Insbesondere als "glutaminhaltige Substanzen", "Glutamin-Derivate" oder "glutaminhaltige Glutaminersatzstoffe" werden Peptide oder Oligopeptide aus D- oder L-Glutamin gemeint (Roth, E. et.al., In vitro cellular & developmental biology, 1988, 24(7): 696-698). Beispielsweise kann Glutamin mit glutaminbasierten Dipeptiden zum Beispiel Alanyl-Glutamin (Ala-Gln) oder Glycyl-Glutamin (Gly-Gln) ersetzt sein (Christie, A. und Butler, M., Journal of Biotechnology, 1994, 37(3):277-90; Sanfeliu, A. und Stephanopoulus, G., Biotechnol. und Bioeng., 1999, 64(1): 46-53; (Kurano, N., et.al., J. Biotechnology, 1990, 15: 113-128). Der Begriff "glutaminhaltige Substanzen" ist auch in der WO 03/106661 (Seite 7, Zeile 1) beschrieben.

Als "Asparagin-Ersatzstoffe", "Asparagin-Derivate" oder "asparaginhaltige Asparaginersatzstoffe" werden Substanzen gemeint, die D- oder L-Asparagin, insbesondere L-Asparagin in einem Molekül beinhalten. Dieses Molekül kann das D- oder L-Asparagin in verschiedenster Art und Weise beinhalten, beispielsweise aber nicht limitiert kann Asparagin mit einem anderen Substanz kovalent gebunden sein. Insbesondere als "Asparagin-Ersatzstoffe", "Asparagin-Derivate" oder "asparaginhaltige Asparaginersatzstoffe" werden Peptide oder Oligopeptide aus D- oder L-Asparagin gemeint. Zum Beispiel aber nicht limitiert Leu-Asn (Sigma, Katalognummer: L0641), oder Ile-Asn (Sigma Katalognummer: I3635), oder Glu-Asn-Gly (Sigma, Katalognummer: P5148). Die Peptide können grundsätzlich 2 oder mehr als 2 Aminosäuren beinhalten.

Als "Glutamat-Ersatzstoffe", "Glutamate-Derivate" oder "glutamathaltige Glutamat-Ersatzstoffe" werden Substanzen gemeint, die D- oder L-Glutamat, insbesondere L-Glutamat in einem Molekül beinhalten. Dieses Molekül kann das D- oder L-Glutamat in verschiedenster Art und Weise beinhalten, beispielsweise aber nicht limitiert kann Glutamat mit einem anderen Substanz kovalent gebunden sein. Insbesondere als "Glutamat-Ersatzstoffe", "Glutamat-Derivate" oder "glutamathaltige Glutamat-Ersatzstoffe" werden Peptide oder Oligopeptide aus D- oder L-Glutamat gemeint. Zum Beispiel aber nicht limitiert Asp-Glu (Sigma, Katalognummer: A1916-250MG), Glu-Glu (Sigma, Katalognummer: G3640-25MG), Glu-His (Sigma, Katalognummer: G6882-100MG), Glu-Leu (Sigma, Katalognummer: G7007-10MG), Glu-Lys (Sigma, Katalognummer: G5136-25MG). Die Peptide können grundsätzlich 2 oder mehr als 2 Aminosäuren beinhalten.

Als "Lactat-Ersatzstoffe", oder "lactathaltige Lactat-Ersatzstoffe" oder "Lactat-Derivate" werden Substanzen gemeint, die D- oder L-Lactat, in einem Molekül beinhalten, zum Beispiel aber nicht limitiert Iron (II) lactate hydrate (Sigma, Katalognummer: 44953), oder Manganase (II) lactate trihydrate (Sigma, Katalognummer: 13227) oder Lactose. Das Molekül kann das D- oder L-Lactat in verschiedenster Art und Weise beinhalten, beispielsweise aber nicht limitiert kann Lactat mit einem anderen Substanz gebunden sein, zum Beispiel aber nicht limitiert (+) Ethyl D-Lactate (Sigma, Katalognummer: 69796). Lactat-Derivate sind auch Moleküle, die leicht intrazellulär in Lactat umgewandelt werden können, zum Beispiel aber nicht limitiert Pyruvat oder Alanin. In diesem Falle kann Pyruvat ins Nährmedium zugegeben werden. Extrazelluläre Pyruvat wird leicht in die Zelle transportiert und durch Pyruvat-Dehydrogenase in Lactat umgewandelt. Im Falle Alanin, kann exrazelluläres Alanin in die Zelle transportiert werden und durch Alanintransaminase über Pyruvat-Zwischenstufe in Lactat umgewandelt werden. Dies gilt auch für alaninhaltige Peptide, daher werden alaninhaltige Peptide ebenfalls als "Lactat-Derivate" definiert.

Der Begriff "Splitverhältnis" auch "Verdünnungsfaktor" genannt, ist ein Maßstab um das Splitverhältnis zweier Flüssigkeiten auszudrücken. Wenn zwei Flüssigkeiten miteinander gemischt werden sollen, damit eine Zielkonzentration erreicht wird, werden die Lösungen im Verhältnis zueinander mit einem Splitverhältnis gemischt. Splitverhältnis wird Anhand folgendes Beispiel erläutert: Eine Zellkultur wurde in einem Kulturgefäß mit einer Zellkonzentration von 2 x 10⁵/ml angeimpft und 3 Tage lang wachsen lassen. Am dritten Tag wurde die Zellkonzentration von 20 x 10⁵/ml gemessen. Diese Kultur wird mit frischem Medium so verdünnt, damit die Zielanimpfkonzentration von 2 x 10⁵/ml wieder erreicht wird. Man muss in diesem Falle die ausgewachsene Kultur (Inokulum) um 1:10 mit frischem Medium verdünnen. Wenn man in der neuen Kultur ein Arbeitsvolumen von 1 I haben soll, muss man 100 ml Inokulum und 900 ml frisches Medium nehmen. In diesem Falle hätte man ein Splitverhältnis von 1:10.

Alle Prozessvarianten, in denen Zellen kultiviert werden, um ein gewünschtes Polypeptid, Protein oder einen gewünschten Impfstoff zu produzieren, sind grundsätzlich für die Applikation der Erfindung geeignet. Ein gemäß der Erfindung bevorzugtes Verfahren ist aber ein Hochzelldichteverfahren. Das Hochzelldichte-Zellkultur-Verfahren ist definiert als ein Verfahren, in dem in einer bestimmten Periode der Kultivierung eine Lebendzellkonzentration von mindestens 1x10⁵/ml, bevorzugt mindestens 1x10⁶/ml erreicht wird, wobei die Kultur von einer einzigen Zelle oder von einer anderen Zellkultur oder von einem Inokulum expandiert werden kann. Gemäß der Erfindung bevorzugte Verfahrensweisen sind kontinuierliche Kultur-, Fed-batch-, Batch-, Perfusion, oder Split-batch-Verfahren.

Die Kultivierung wird vorzugsweise in Batch-Verfahren durchgeführt, wobei alle Komponenten für Zellkultivierung am Beginn des Prozesses im Kulturgefäß vorgelegt werden. In Batch-Verfahren werden die Zellen in einem geeigneten Medium angeimpft und nach einer gewissen Kultivierungszeit geerntet. Anschließend wird das gewünschte Produkt, zum Beispiel ein Polypeptid, aus der Kultur isoliert.

Ein gemäß der Erfindung besonders bevorzugtes Verfahren ist ein Fed-batch-Verfahren, in dem die Zellen zwischen dem Animpfen und dem Ende des Prozesses mit einem weiteren Nährmedium, beispielsweise mit einem Feedmedium kontaktiert werden. Fed-batch Prozess startet beispielsweise aber nicht limitiert mit einem Basalmedium. In Basalmedium werden die Zellen angeimpft. Nach einer gewissen Zeit, beispielsweise aber nicht limitiert 1-4 Tage nach Animpfen wird die Kultur mit einem weiteren Nährmedium kontaktiert, beispielsweise kann dieses Nährmedium ein Feedmedium (Zufütterungsmedium) sein. Das Zufütterungsmedium kann batchweise, in Intervallen oder kontinuierlich über eine eingestellte Dosis in die Zellkultur zugegeben werden. Somit steigt das Volumen der Kultur während der Kultivierung an.

Eine weitere, gemäß der Erfindung geeignete Kultivierungsmethode ist die Perfusion, in dem die Zellen nach einer Wachstumsphase in einem Kulturmedium mit weiterem frischen Medium zum Beispiel mit Perfusionsmedium versorgt werden, während das verbrauchte Medium mit oder ohne Zellen aus dem Prozess abgezogen wird. Perfusionsmedien können sich von Kulturmedien (Basalmedien) unterscheiden, oder das gleiche Basalmedium kann auch als Perfusionsmedium eingesetzt werden. In Perfusionsverfahren werden die Zellen zuerst in ein Basalmedium inokuliert. Im Laufe der Zellkultivierung werden die Zellen (Biomasse) von der Zellkultur (Zellsuspension) getrennt und einerseits das verbrauchte Medium aus dem Prozess abgezogen, andererseits werden den Zellen neue Nährstoffe durch frisches Medium zur Verfügung gestellt. Wenn während des Prozesses Zellen im Kultursystem zurückgehalten werden, wird dies "Perfusion" genannt. Wenn während des Prozess Zellen aus dem System mit dem verbrauchten Medium mit entfernt werden, wird dies "kontinuierliches Verfahren" genannt. In Perfusionsprozessen können die Zellen auch in bestimmten Zeitabständen aus dem Kultivierungssystem entfernt werden um eine maximale Zellkonzentration einhalten zu können. Dieser Vorgang ist als "bleeding" dem Fachmann bekannt. Die Zelltrennung erfolgt mit unterschiedlichen Technologien, wobei einige Technologien die Zelltrennung indirekt fördern. Beispiele für einige mögliche Zelltrennverfahren sind Filtration, Zellenkapsulierung, Zelladhärenz an Mikrocarriern, Zellsedimentation oder Zentrifugation.

Ein weiteres, gemäß der Erfindung geeignetes Verfahren ist das Split-batch-Verfahren (wiederholter Satzweise Betrieb) mit oder ohne Zufütterung der Zellen. In diesem Verfahren wird nach einer Wachstumsphase ein Teil der Zellsuspension geerntet und der Rest der Zellsuspension wird im Fermenter als Inokulum des nächsten Satzbetriebs gelassen. Der Fermenter wird mit diesem Inokulum anschließend wieder bis zum gewünschten Arbeitsvolumen mit frischem Medium befüllt und somit startet der zweite Satzweise-Betrieb.

Die Entfernung von Proben aus dem Kultivierungssystem für offline Messungen während oben genannter Verfahrensweisen hat mit den beschriebenen Kultivierungsverfahren nichts zu tun. Es dient lediglich dazu um den Prozess besser zu verstehen oder zu kontrollieren.

Der Begriff "Ernte" oder "Zellernte" ist ein Hinweis für das Ende eines Produktionsbatches in Upstream-Prozess. In Batch, und Fed-batch-Prozess kann der Upstreamprozess nur mit einer Ernte enden, während es in Split-batch, Perfusion- und in kontinuierlichen Verfahren mehrere Ernten geben kann. Je nach ausgewählter Verfahrensweise kann der Zellkultivierungsprozess (Upstreamprozess) eine Lag-Phase, eine Exponentiellephase, eine Stationärephase und eine Absterbephase beinhalten. Der Fermentationsprozess kann in einer dieser Wachstumsphasen beendet werden. Bevorzugt wird der Fermentationsprozess in der stationären Phase oder in Absterbephase beendet. Am Ende des Upstream-Prozesses werden die Zellen von der Suspension getrennt. Dieser Zelltrennungsprozess wird hier "Ernte" oder "Zellernte" genannt. Nach der Zellernte erfolgen weitere Aufreinigungsschritte um das Produkt isolieren zu können.

Der Begriff "Inokulation" bedeutet das Animpfen eines neuen Kultivierungssystems (zum Beispiel eines Bioreaktors und eines geeigneten Nährmediums, insbesondere eines Zellkulturmediums) mit Zellen. Das Inokulum, d.h. die Zellen oder die Zellsuspension werden im Voraus für diesen Zweck vorbereitet. Üblicher Weise werden die zu inokulierenden Zellen vor dem eigentlichen Produktionsprozess expandiert, sodass man genug Zellen hat, um das neue Kultivierungssystem mit gewünschter Zellkonzentration animpfen zu können.

Mit Zellwachstum ist eine Zunahme der Lebendzellkonzentration mindestens in einer der Kultivierungsphasen gemeint. Der Begriff "Zellvitalität" drückt das Verhältnis der Lebendzellkonzentration zu der Gesamtzellkonzentration aus, welches auch die toten Zellen umfasst. Dieses Verhältnis kann auf eine beliebiger Art und Weise berechnet werden. Eine Methode ist die Markierung der Zellen mit einem Farbstoff und somit die Differenzierung der lebenden und toten Zellen. Ein Beispiel zur Unterscheidung der toten und lebenden Zellen ist der Einsatz von Tyrpanblau-Farbstoff. Die markierten Zellen werden anschließend gezählt und die Zahl der toten und lebenden Zellen bestimmt. Die Zellvitalität wird kalkuliert, in dem das Verhältnis zwischen der Lebendzellzahl und der Gesamtzellzahl gebildet wird.

Der Begriff "Osmolalität" ist ein Maßstab für den osmotischen Druck der gelösten Partikel in einer wässrigen Lösung. Die gelösten Partikel beinhalten sowohl die Ionen als auch die nicht-ionisierten Moleküle. Osmolalität ist ausgedrückt als die Konzentration der osmotisch aktiven Partikel, gelöst in 1 kg Wasser (1 mOsmol/kg H₂O bei 38°C ist äquivalent zu einem osmotischen Druck von 19 mm Hg). Der Begriff "Osmolarität" bedeutet, die Anzahl der Partikel, die in 1 I Lösung gelöst sind. Substanzen, die in Kulturmedien vorkommen und die die Osmolalität der Lösung erhöhen, sind daher beispielsweise Proteine, Peptide, Animosäuren, nicht-metabolisierbare Polymere, Vitamine, Ionen, Salze, Zucker, Metabolite, organische Säuren, Lipide, etc.

Der Begriff "pH-Wert" ist der negative dekadische Logarithmus des Zahlenwertes der molaren Wasserstoffionenaktivität a_{H}⁺ (pH = -log a_{H}⁺). Lösungen mit einem pH-Wert kleiner 7,0 reagieren sauer, mit einem pH-Wert von 7,0 sind sie neutral. Bei Lösungen mit einem pH-Wert größer als 7,0 reagieren sie basisch. Wenn pH-Werte zweier Lösungen miteinander verglichen wird, ist die Lösung mit höherem pH-Wert definiert als "basischer" im Vergleich zu der Lösung mit niedrigerem pH-Wert, z.B. Lösung 1 hat einen pH-Wert von 6,8, Lösung 2 hat einen pH-Wert von 7,0. In diesem Falle ist die Lösung 2 basischer im Vergleich zu Lösung 1. pH-Werte der Herstellverfahren können in unterschiedlicher Art und Weise gemessen werden. Beispielsweise aber nicht limitiert, kann der pH-Wert eines Bioreaktors on-line mit einer pH-Elektrode gemessen werden. Eine andere Methode den pH-Wert der Kultur zu bestimmen ist eine off-line Probenahme und Detektion des pH-Wertes mit einer externen pH-Elektorde.

Weitere vorteilhafte Ausgestaltungen sind den Unteransprüchen zu entnehmen.

Die Erfindung wird anhand folgender Beispiele näher erläutert.

Die Figuren zeigen:
Figur 1: Wachstumsdaten von metabolisch optimierten CHO-Zellen in einem offenbarten Nährmedium ohne Glucose und ohne Glutamin unter Routine-Stammhaltung.
Figur 2: Wachstumskurve von Zellen in offenbarten Nährmedium im Vergleich zu einer Kontrolle in Fed-batch Verfahren.
Figur 3: Verlauf der Lactat-Konzenration während einer Fed-batch-Kultivierung.
Figur 4: Ammonium-Bildung während einer Fed-batch-Kultivierung.
Figur 5: Verlauf des pH-Wertes eines Kultivierungs-Prozesses. Die pH-Werte wurden off-line gemessen und während der Kultivierung wurde der pH-Wert des Prozesses nicht geregelt.
Figur 6: Wachstumsverhalten von Wildtypzellen (metabolisch nicht optimiert) in einer Fed-batch-Kultivierung mit offenbarten Basalmedien und Feedmedien in unterschiedliche Kombination.
Figur 7: Wachstumsverhalten von metabolisch optimierten Zellen in einer Fed-batch-Kultivierung in offenbarten Basalmedium und mit 2 unterschiedlichen Feedmedien.
Figur 8: Wachstumsverhalten von metabolisch optimierten Zellen in einer Fed-batch-Kultivierung in offenbarten Nährmedien mit und ohne Natrium-Succinat.
Figur 9: Verlauf der Lactatkonzentration in offenbarten Nährmedien mit und ohne Natrium-Succinat in einer Fed-batch-Kultivierung.

### Beispiele:

### Material und Methoden

### Zellkulturbedingungen

Zellen wurden in der Gasphase von flüssigem Stickstoff gelagert. Ein bis zwei Ampullen wurden in 37°C vorgewärmten Nährmedium aufgetaut und in Spinnerflaschen, in Schüttelkolben oder in T-Falschen transferiert. In den ersten Kultivierungstagen erfolgte je nach Klon ein Mediumwechsel.

Zellen wurden je nach Klon entweder jeden zweiten Tag oder jeden dritten Tag oder nach abwechselnd zwei und drei Tagen in neues Medium gesplittet, d.h. ein Teil der Zellsuspension wurde der Kultur entnommen und ein kleiner Teil der Zellsuspension wurde als Inokulum der nächsten Kultur weiterverwendet. Dieses Inokulum wurde mit frischem vorgewärmten Nährmedium aufgestockt. Zellen wurden bei 37°C in einem Brutschrank bei einer CO₂-Atmosphäre inkubiert. Eine Alternative, um Zellen zu inkubieren war die Kultivierung in einem Brutraum bei 37°C, Zellen wurden in dieser Art mehrere Passagen lang in Kultur gehalten. Mit einer Passage ist eine Kultivierungsdauer von zwei bis drei Tagen gemeint. Zu unterschiedlichen Zeitpunkten wurde aus der Stammhaltung Zellsuspension genommen und als Inokulum für Experimente verwendet. Die Experimente wurden in diversen Kultivierungssystemen durchgeführt, zum Beispiel in Spinnerflaschen, T-Flaschen, Schüttelkolben, Kulturröhrchen oder in geregelten Bioreaktoren.

Für die Experimente wurde eine CHO Zelllinie benutzt, welche einen Antikörper exprimiert. Die Produktkonzentration (Antikörperkonzentration) im Zellüberstand wurde durch ELISA bestimmt.

Ein CHO-Medium von der Firma Sigma (CR 1020, Lot: 64K2403) wurde für die Experimente als Nährmedium (Basalmedium) verwendet. Das ist ein serumfreies, proteinfreies Nährmedium. Es handelt sich um ein fertig hergestelltes und steril filtriertes Nährmedium.

Die Experimente wurden entweder in Batch-Verfahren oder in Fed-batch-Verfahren durchgeführt. Wenn der Prozess im Fed-batch-Verfahren durchgeführt wurde, wurde der Prozess zuerst in der Batch-Verfahrensweise in oben genanntem Basalmedium gestartet und Zellen wurden 1-4 Tage in dieser Weise kultiviert. Nach 1-4 Tagen wurden die Zellen mit einem Feedmedium zugefüttert, d.h. der Prozess wurde auf Fed-batch umgestellt. Die Zufütterung erfolgte in regulären Abständen. Bei Fed-batch Experimenten wurde ein proteinfreies und serumfreies Feedmedium von der Firma HyQ (R05.2 supplement, Cat. Nr: SH30584.04, Lot Nr: APF21457G) verwendet.

Alle verwendeten hier offenbarten Substanzen (Chemikalien) wurden von der Firma Sigma erhalten und daraus Stammlösungen vorbereitet. Aus den Stammlösungen wurden die Substanzen zu der gewünschten Endkonzentration in Kulturröhrchen gegeben. Die so vorbereiteten unterschiedlichen Kulturröhrchen wurden zum Schluss mit dem Antikörper produzierenden CHO-Klon mit einer Ziel-Animpfzellkonzentration (1-3 x 10⁵/ml) angeimpft. Dazu wurde die entsprechende Menge an Inokulumszellen kalkuliert und die benötigte Menge an Inokulums-Zellsuspension zentrifugiert. Das Zellpellet wurde zum Schluss in dem entsprechenden Volumen von frischem Nährmedium ohne hier offenbarten Substanzen (Sigma, CR 1020, Lot: 64K2403) aufgenommen. Dadurch wurde in der zu untersuchenden Zellsuspension das gewünschte Animpfzellkonzentration eingestellt. Die so vorbereitete Zellsuspension wurde in die Kulturröhrchen proportioniert, die zuvor mit den erfindungsgemäßen Substanzen versehen waren. Die Kulturröhrchen wurden bei 37°C, und 8 %iger CO₂ Atmosphäre unter Schütteln inkubiert. Die Kulturröhrchen wurden 8-11 Tage lang im Batch-Verfahren oder im Fed-batch-Verfahren kultiviert. Aus den Kulturgefäßen wurden in regulären Abständen, zum Beispiel täglich oder bis jeden dritten Tag, Proben genommen um Offline-Parameter zu messen. Aus den Proben wurden die metabolischen Parameter und Zellkonzentrationen gemessen. Die Zellkonzentration und die Zellvitalität wurden mit einem CeDex-Gerät gemessen. Die metabolischen Parameter (Glucose, Lactat, Glutamin und Ammonium) wurden mit einem Analyser (BioProfile 100) bestimmt.

### Beispiel 1: Batch-Experiment mit den erfindungsgemäßen Substanzen

Folgende Endkonzentration der offenbarten Substanzen wurden in einem Nährmedium nach der Inokulation eingestellt:
Natrium-Succinat mit 1 g/l Endkonzentration in Nährmedium (erfindungsgemäß)
Äpfelsäure mit 1 g/l Endkonzentration in Nährmedium
α-Keto-Glutarat mit 1 g/l Endkonzentration in Nährmedium
Natrium-Fumarat mit 1 g/l Endkonzentration in Nährmedium
Weinsäure mit 0,5 g/l Endkonzentration in Nährmedium
Adipinsäure mit 0,5 g/l Endkonzentration in Nährmedium
Natrium-Lactat mit 1 g/l Endkonzentration in Nährmedium
Ornithin mit 1 g/l Endkonzentration in Nährmedium
Citrullin mit 1 g/l Endkonzentration in Nährmedium
Natrium-Pyruvat mit 0,5 g/l Endkonzentration in Nährmedium

Die offenbarten Substanzen wurden im Batch-Verfahren getestet. Das Nährmedium oder Basalmedium (Serum freies, Protein freies CHO Medium von der Firma Sigma CR 1020, Lot: 64K2403) hatte eine Glucosekonzentration von 4,7 g/l und eine Glutaminkonzentration von 1,5 mM (Tabelle 1).

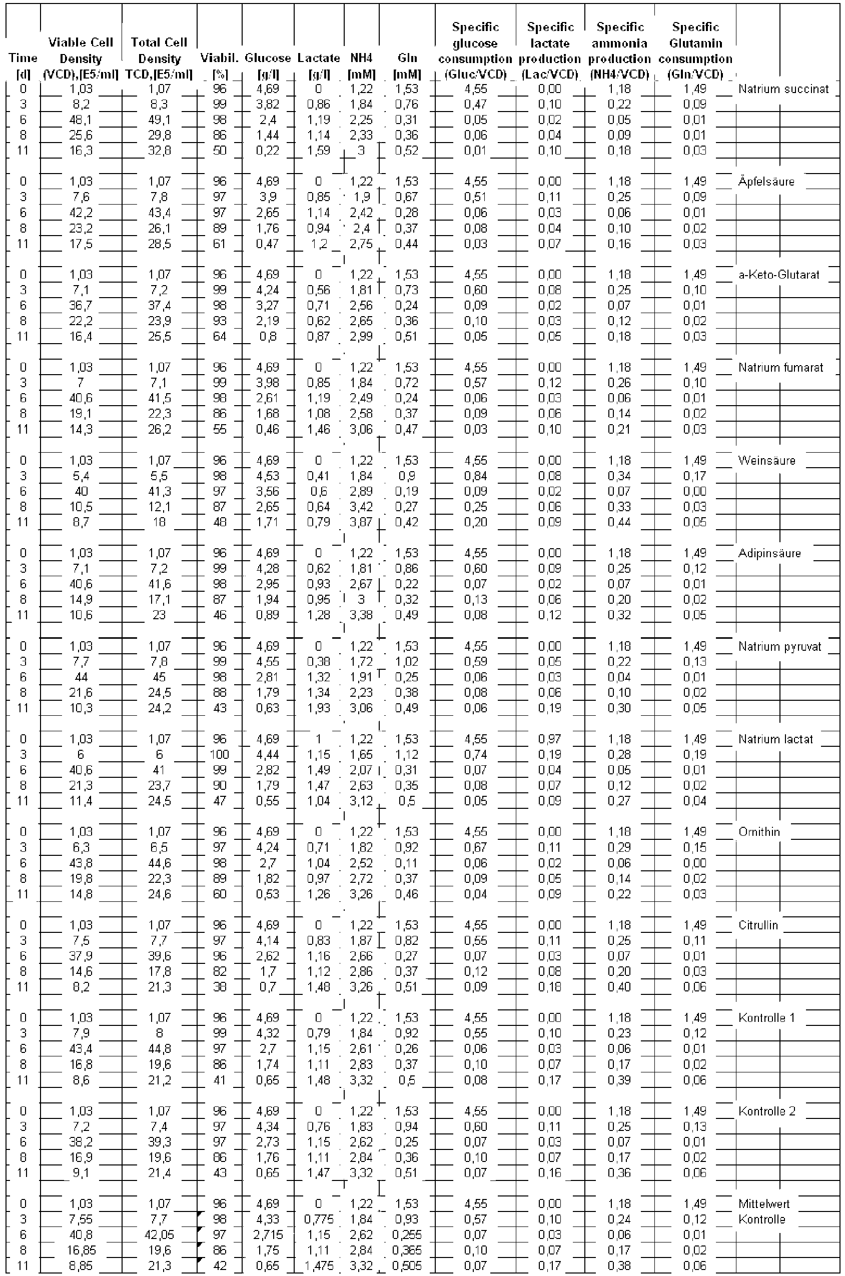

### Tabelle 1: Ergebnisse des Batch-Experimentes mit den offenbarten Substanzen

### Beispiel 2: Fed-batch-Experiment mit den offenbarten Substanzen

Folgende Endkonzentration der offenbarten Substanzen wurden in einem Nährmedium nach der Inokulation eingestellt:
Natrium-Succinat mit 1 g/l Endkonzentration in Nährmedium (erfindungsgemäß)
Äpfelsäure mit 1 g/l Endkonzentration in Nährmedium
α-Keto-Glutarat mit 1 g/l Endkonzentration in Nährmedium
Natrium-Fumarat mit 1 g/l Endkonzentration in Nährmedium
Weinsäure mit 0,5 g/l Endkonzentration in Nährmedium
Adipinsäure mit 0,5 g/l Endkonzentration in Nährmedium
Natrium-Lactat mit 1 g/l Endkonzentration in Nährmedium
Ornithin mit 1 g/l Endkonzentration in Nährmedium
Natrium-Pyruvat mit 0,5 g/l Endkonzentration in Nährmedium

Als Unterschied zu Beispiel 1 wurden die offenbarten Substanzen im Fed-batch Verfahren getestet und aus jeder zu testendem Substanz wurden zwei unabhängige Kulturröhrchen inokuliert (n=2).

Das Nährmedium (Serum freies, Protein freies CHO Medium von der Firma Sigma, CR 1020, Lot: 64K2403) hatte eine Glucosekonzentration von 4,7 g/l und eine Glutaminkonzentration von 0,7 mM. Das verwendete Feedmedium war von der Firma HyQ, (R05.2 supplement, Cat. Nr: SH30584.04, Lot Nr: APF21457G). Das Feedmedium war ohne Glutamin (Tabelle 2).

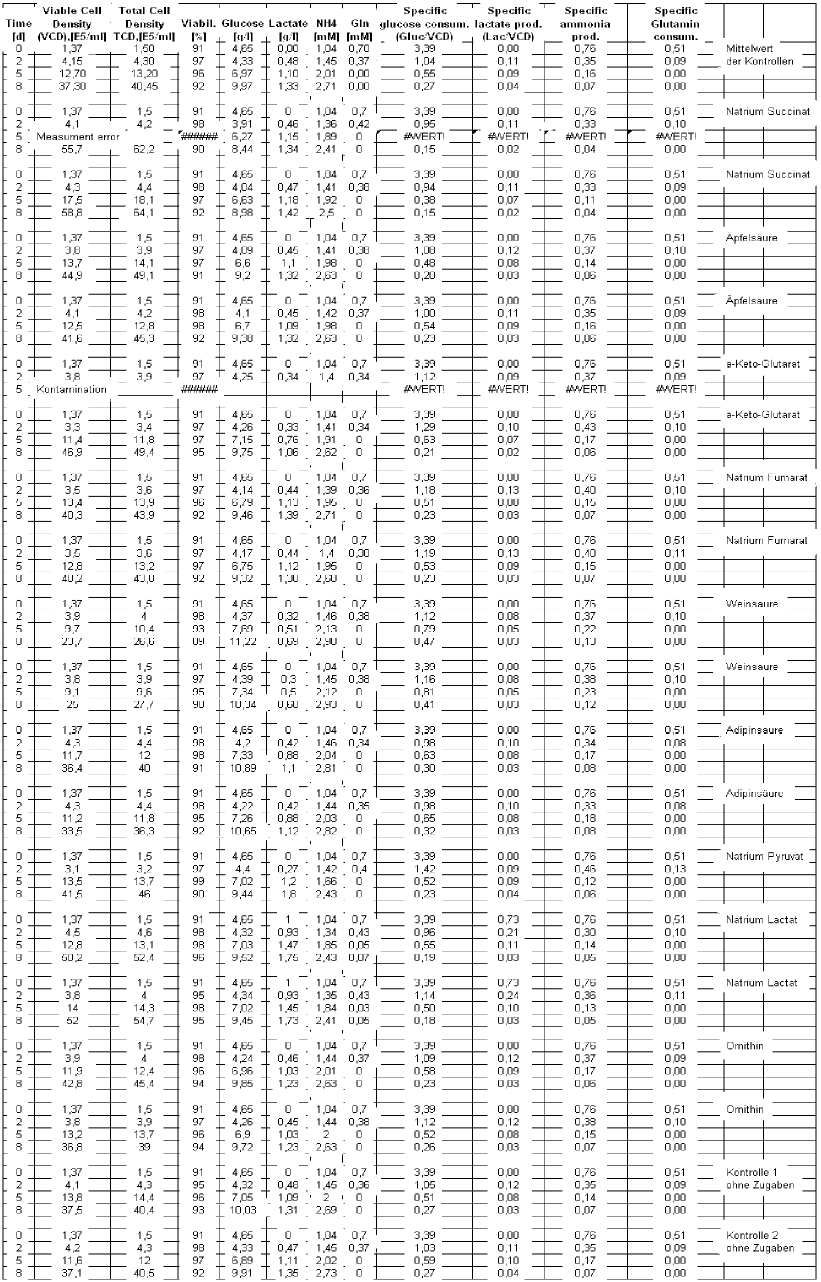

### Tabelle 2: Ergebnisse des Fed-batch-Experimentes mit den offenbarten Substanzen

Aus ausgewählten Proben wurde die Produktkonzentration (Antikörperkonzentration) im Überstand durch ELISA bestimmt (Tabelle 3)

**Tabelle 3: Ergebnis der Produktbestimmung der ausgewählten Substanzen. Der absolute Wert der Kontrolle wurde auf 100 % gesetzt.**

| | Antikörperkonzentration [%] |
|---|---|
| Kontrolle ohne Zugaben | 100 |
| Natrium-Succinat B | 127 |
| Natrium-Lactat B | 124 |

### Beispiel 3: Fed-batch Experiment mit den offenbaren Substanzen und ohne Glutamin in allen eingesetzten Kulturmedien

Folgende Endkonzentration der erfindungsgemäßen Substanzen wurden in einem Nährmedium nach der Inokulation eingestellt:

### Kontrolle, ohne Zugaben

Natrium-Succinat mit 1 g/l Endkonzentration in Nährmedium (erfindungsgemäß)
α-Keto-Glutarat mit 1 g/l Endkonzentration in Nährmedium
Natrium Lactat mit 0,5 g/l Endkonzentration in Nährmedium

Die offenbarten Substanzen wurden unter Fed-batch-Bedingungen getestet. Das Nährmedium (Serum freies, Protein freies CHO Medium von der Firma Sigma, CR 1020, Lot: 64K2403) hatte eine Glucosekonzentration von 4,7 g/l und es war glutaminfrei. Das verwendete Feedmedium war von der Firma HyQ, (R05.2 supplement, Cat. Nr: SH30584.04, Lot Nr: APF21457G). Das Feedmedium war ohne Glutamin (Tabelle 4).

**Tabelle 4: Ergebnisse des Fed-batch-Experimentes mit den offenbarten Substanzen in glutaminfreien Kulturmedien**

| **Time [d]** | **VCD [% of control]** | **TCD [% of control]** | **Viabil. [%]** | **Sp. Gluc cons.** | **Sp. Lactate prod. [% of control]** | **Sp. NH4 prod [% of control]** | |
|---|---|---|---|---|---|---|---|
| | | | | | | | Kontrolle |
| 0 | 100 | 100 | 94 | 100 | 100 | 100 | ohne Gln |
| 3 | 100 | 100 | 89 | 100 | 100 | 100 | |
| 5 | 100 | 100 | 91 | 100 | 100 | 100 | |
| 7 | 100 | 100 | 88 | 100 | 100 | 100 | |
| 9 | 100 | 100 | 93 | 100 | 100 | 100 | |
| | | | | | | | |
| | | | | | | | Natrium Succinat |
| 0 | **100** | **100** | 94 | 95 | - | **98** | Ohne Gln |
| 3 | **132** | **124** | 95 | 74 | **75** | **72** | |
| 5 | **111** | **108** | 95 | 88 | **93** | **90** | |
| 7 | **117** | **118** | 87 | 78 | **89** | **85** | |
| 9 | **136** | **134** | 94 | 69 | **78** | **74** | |
| | | | | | | | alfa-Keto Glutarat |
| 0 | 100 | 100 | 94 | 94 | - | 100 | ohne Gln |
| 3 | 103 | 94 | 96 | 98 | 70 | 94 | |
| 5 | 104 | 103 | 93 | 98 | 72 | 94 | |
| 7 | 99 | 96 | 90 | 95 | 72 | 95 | |
| 9 | 92 | 92 | 93 | 109 | 79 | 103 | |
| | | | | | | | |
| | | | | | | | Natrium Lactat |
| 0 | **100** | **100** | 94 | **93** | **-** | **98** | ohne Gln |
| 3 | **148** | **140** | 94 | **66** | **72** | **63** | |
| 5 | **136** | **128** | 97 | **74** | **66** | **72** | |
| 7 | **147** | **136** | 95 | **62** | **57** | **66** | |
| 9 | **133** | **131** | 95 | **71** | **65** | **73** | |

Beispiel 4: Fed-batch Experiment mit den offenbarten Substanzen und mit glutaminhaltigen Glutaminersatzstoffen

Folgende Endkonzentration der offenbarten Substanzen wurden in einem Nährmedium nach der Inokulation eingestellt:
Kontrolle, ohne Zugaben
Natrium-Succinat mit 1 g/l Endkonzentration in Nährmedium (erfindungsgemäß)
α-Keto-Glutarat mit 1 g/l Endkonzentration in Nährmedium
Natrium Lactat mit 0,5 g/l Endkonzentration in Nährmedium

Die offenbarten Substanzen wurden unter Fed-batch-Bedingungen getestet. Das Nährmedium (Serum freies, Protein freies CHO Medium von der Firma Sigma, CR 1020, Lot: 64K2403) hatte eine Glucosekonzentration von 4,7 g/l und eine Glutaminkonzentration von 4 mM. Das verwendete Feedmedium war von der Firma HyQ, (R05.2 supplement, Cat. Nr: SH30584.04, Lot Nr: APF21457G). Das Feedmedium war mit 3 g/l Alanyl-Glutamin (Ala-Gln) und mit 3 g/l mit Glycyl-Glutamin (Gly-Gln) versetzt. Das Feedmedium war ohne Glutamin (Tabelle 5).

**Tabelle 5: Ergebnisse des Fed-batch-Experimentes mit den offenbarten Substanzen und mit Glutamin-Derivaten**

| Time [d] | Viable Cell Density (VCD),[E5/ml] | Total Cell Density (TCD),[E5/ml] | Viabil. [%] | | |
|---|---|---|---|---|---|
| | | | | Kontrolle | |
| 0 | 1,0 | 1,1 | 96 | | |
| 3 | 7,9 | 8,2 | 96 | | |
| 5 | 40,4 | 41,7 | 97 | | |
| 7 | 21,0 | 37,2 | 56 | | |
| 9 | 13,6 | 33,2 | 41 | | |
| | | | | | |
| | | | | Natrium Succinat | |
| 0 | 1,0 | 1,1 | 96 | | |
| 3 | 8,8 | 9,3 | 95 | | |
| 5 | 54,3 | 56,7 | 96 | | |
| 7 | 34,5 | 42,0 | 82 | | |
| 9 | 26,7 | 35,9 | 74 | | |
| | | | | | |
| | | | | alfa-Keto Glutarat | |
| 0 | 1,0 | 1,1 | 96 | | |
| 3 | 7,1 | 7,6 | 93 | | |
| 5 | 38,5 | 40,1 | 96 | | |
| 7 | 30,2 | 36,0 | 84 | | |
| 9 | 21,0 | 31,2 | 67 | | |
| | | | | | |
| | | | | Natrium Lactat | |
| 0 | 1,0 | 1,1 | 96 | | |
| 3 | 7,6 | 7,9 | 96 | | |
| 5 | 43,3 | 45,2 | 96 | | |
| 7 | 34,3 | 41,1 | 83 | | |
| 9 | 25,2 | 34,7 | 73 | | |

Beispiel 5: Routinekultivierung der metabolisch optimierten Zellen in Stammhaltung mit offenbarten Substanzen in einem Glucosefreien und Glutaminfreien Nährmedium (Basalmedium)

Ein glukosefreies und glutaminfreies kommerzielles Nährmedium wurde besorgt. Dieses Medium wurde gemäß Tabelle 6 mit offenbarten Substanzen aufgestockt und somit das endgültige Nährmedium vorbereitet. Die in der Tabelle 6 dargestellte Konzentration der Substanzen ist die Endkonzentration der jeweiligen Substanzen in Medium. Dieses Nährmedium entspricht einem serumfreien, proteinfreien, peptonfreien Medium mit erfindungsgemäßen Substanzen. Das endgültige Nährmedium für Zellkultivierung ist also, glucosefrei, glutaminfrei, beinhaltet Galactose und beinhaltet Lactat.

**Tabelle 6: Zusammensetzung des Nährmediums (Basalmedium) für die Routinekultivierung der CHO-Zellen mit offenbarten Substanzen in Stammhaltung**

| | | **Glukose** | **Galaktose** | **Lactat** | **Glutamin** | **Glutamat** | **Asparagin** | **Na-Succinat** |
|---|---|---|---|---|---|---|---|---|
| | | **[g/l]** | **[g/l]** | **[g/l]** | **[g/l]** | **[g/l]** | **[g/l]** | **[g/l]** |
| **MEL** | Basalmedium | 0 | 3 | 2,5 | 0 | 0,8 | 0,23 | 0 |

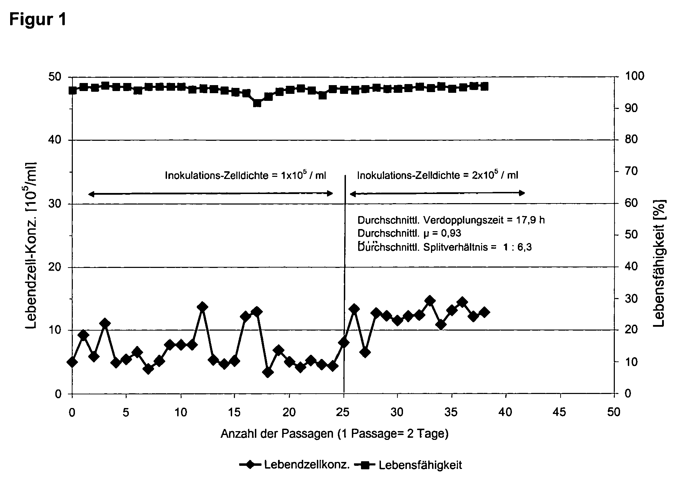

Die metabolisch optimierten CHO-Zellen wurden in Schüttelkolben unter Batchbedingungen für Stammhaltung kultiviert. Zellen wurden in obengenantem Nährmedium mit einer Animpfzellkonzentration von 1-2 x 10⁵ Lebendzellen pro ml angeimpft. Die Kultur wurde bei 37 °C und bei 7,5 % pCO₂ Atmosphäre 2 Tage lang inkubiert und am Tag 2 wurde die Kultur subkultiviert. Dazu wurde die Zellkonzentration am Tag 2 gezählt und ein Kultivierungsgefäß mit gleicher Animpfzellkonzentration in gleiches frisches Medium angeimpft. Eine Kultivierungsperiode von zwei Tagen wurde als eine Passage definiert. Die Kultur wurde in dieser Weise mehrere Passage lang kultiviert und die Lebendzellkonzentration bei jeder Passage, kurz vor Subkultivierung bestimmt. Die ermittelten Werte wurden in einem Diagramm aufgetragen (Figur 1). Aus Figur 1 ist ersichtlich, dass die Zellen in dem erfindungsgemäßen Medium gut wachsen können. Zellen haben in dem offenbarten Medium eine Verdopplungszeit von 17,9 Stunden. Zellen verfügen ein hohes Splitverhältnis von 1:6,3 in einem Kultivierungsmodus von 2 Tagen. Wenn Zellen in Stammhaltung jeden dritten Tag gesplittet werden, haben sie ein Splitverhältnis von größer als 1:10. Dieses hohe Splitverhältnis, bzw. niedrige Verdopplungszeit ist erreicht in einem serumfreien, proteinfreien, peptonfreien, glucosefreien, und glutaminfreien Medium.

Ab hier wurde die zweite Gruppe von Fed-batch Experimenten durchgeführt. In allen Experimenten ab hier (Beispiele 6 bis 9) wurden folgende gemeinsame Materialen und Methoden verwendet:
Das erste Nährmedium, das verwendet worden ist, ist ein kommerzielles Nährmedium (Basalmedium). Es handelt sich bei diesem Basalmedium um ein glukosefreies und glutaminfreies Basalmedium, dessen Rezeptur nicht bekannt ist. Dieses Basalmedium wurde in jeweiligen Beispielen mit offenbarten Substanzen aufgestockt. Die angegebene Konzentration der Substanzen in den Beispielen ist die Endkonzentration der jeweiligen Substanz in Basalmedium. Daher wurden gegebenenfalls die Nährmedien mit offenbarten Substanzen soweit aufgestockt um die finale, angegebene Konzentration zu erreichen. Nach der Aufstockung wurde der pH-Wert des Basalmediums auf 7,1 ±0,1 eingestellt. Die Osmolalität des Basalmediums wurde auf 310 ±20 mOsmol/kg H₂O eingestellt. Somit wurde das endgültige Basalmedium vorbereitet und sterilfiltriert. Das so vorbereitete Basalmedium entspricht einem serumfreien, proteinfreien, und peptonfreien Basalmedium.

Das zweite verwendete Nährmedium ist ein Feedmedium (Zufütterungsmedium). Es ist ein Serum freies, proteinfreies Feedmedium. Das Feedmedium ist eine Eigenformulierung, bestehend aus unter anderem Aminosäuren, Salzen und Vitaminen. Dieses Feedmedium wurde in jeweiligen Beispielen mit offenbarten Substanzen aufgestockt. Die angegebene Konzentration der Substanzen in den Beispielen ist die Endkonzentration der jeweiligen Substanz in Feedmedien. Das finale Feedmedium hatte einen pH-Wert von 6,0 bis 7,8 und eine Osmolalität von 460-750 mOsmol/kg H₂O.

Alle Experimente wurden unter fed-batch Bedingungen durchgeführt. Die Experimente wurden gestartet, in dem die Basalmedien mit CHO-Zellen inokuliert worden sind. Die Inokulationszellkonzentration betrug 1-3 10⁵/ml. Die Kulturen wurden bis zu 7 Tage laufen gelassen und während der Kultivierung mit dem korrespondierenden Feedmedium in regulären Abständen zugefüttert.

Die verwendete Terminologie in Experimenten wird Anhand folgender Beispiele erläutert. Name des Experiments ist beispielsweise S4:MEL-St-MEL.

### S4: Nummer des Experiments. Es variiert von S1 bis S6

Erste Abkürzung (MEL): Spezifikation der eingesetzten Zellen. Dies kann entweder MEL (metabolically engineered), oder St (standard, Wildtyp) heißen.

Zweite Abkürzung (St): Spezifikation des eingesetzten Basalmediums. Dies kann entweder MEL (offenbartes Basalmedium), oder St (Standard Basalmedium) heißen.

Dritte Abkürzung (MEL): Spezifikation des eingesetzten Feedmediums. Dies kann entweder MEL (offenbartes Feedmedium), oder St (Standard Feedmedium) heißen.

In den erfindungsgemäßen Verfahren war es notwendig Zellen mit Lactat zu zufüttern, da sie Lactat als Kohlenhydratquelle benötigen. Solche Feedmedien, bzw. die Kulturen, die extra mit Lactat zugefüttert worden sind, wurden in Medientabellen mit dem Vermerk "Extra feed" gekennzeichnet. Dies liegt darin, dass Lactat zu den angegebenen Feedmedien dazu gehört. Dennoch wurde Lactat in die Feedmedien nicht dazugegeben, da man Löslichkeitsprobleme vermeiden wollte. Stattdessen wurde die Lactatkonzentration täglich bestimmt und bei Bedarf, (bevor Lactat limitiert) aus einer getrennten Stammlösung der Kultur dazu gegeben.

### Beispiel 6: Fed-batch Experiment mit den erfindungsgemäßen Substanzen, ohne Glukose und ohne Glutamin in Nährmedien

Für dieses Fed-batch Experiment wurde folgende Medienkombination eingesetzt:

**Tabelle 7: Die Endkonzentration der Substanzen in eingesetzten Nährmedien**

| | **Glukose** | **Galaktose** | **Lactat** | **Glutamin** | **Glutamat** | **Asparagin** |
|---|---|---|---|---|---|---|
| | **[g/l]** | **[g/l]** | **[g/l]** | **[g/l]** | **[g/l]** | **[g/l]** |
| | | | | | | |
| **Basalmedium der Kontrolle S1:St-St-St** | 3,4 | 0 | 0 | 0,8 | 0,15 | 0,23 |
| **Feedmedium der Kontrolle S1:St-St-St** | 60 | 0 | 0 | 13 | 0,15 | 0,15 |
| | | | | | | |
| **Basalmedium des Experiments S1:MEL-MEL-MEL** | 0 | 3 | 2,5 | 0 | 0,8 | 0,23 |
| **Feedmedium des Experiments S1:MEL-MEL-MEL** | 0 | 60 | Extra feeded | 0 | 7,15 | 0,15 |

| | | | | | | |
|---|---|---|---|---|---|---|
| S1:St-St-St = Standardzelle (Wildtypzelle), Standard Basalmedium, Standard Feedmedium S1:MEL-MEL-MEL = Metabolisch optimierte Zelle, offenbartes Basalmedium, erfindungsgemäße Feedmedium | | | | | | |

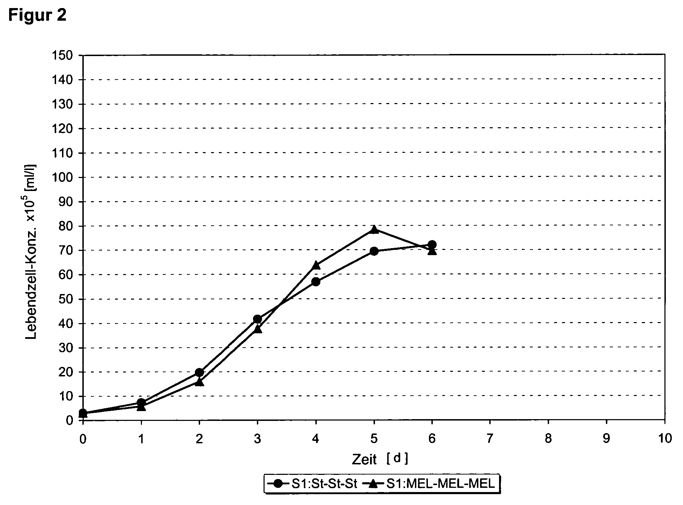

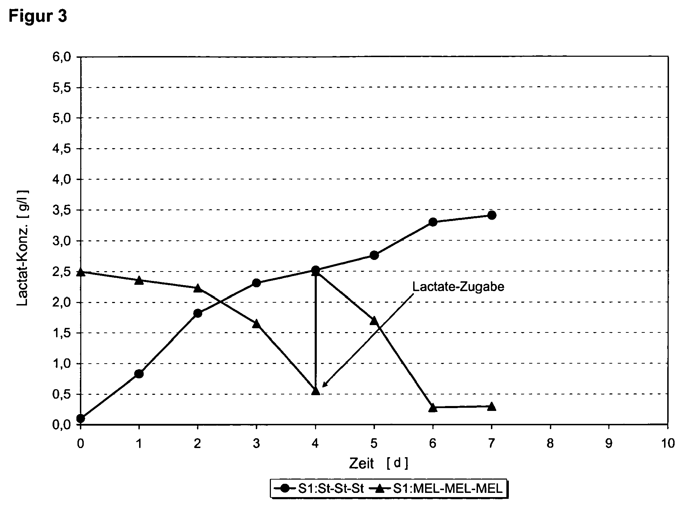

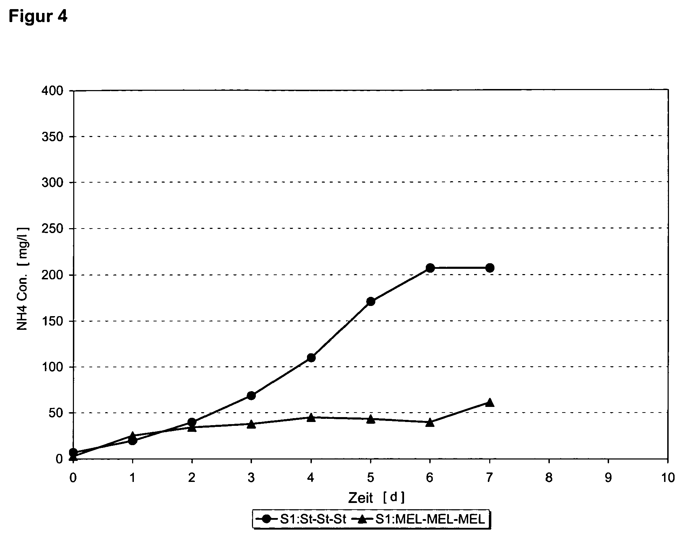

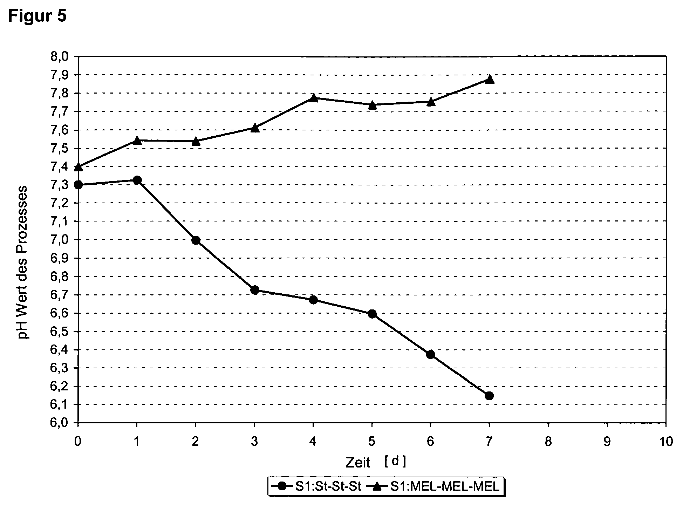

Die Ergebnisse zeigen, dass die metabolisch optimierten Zellen in hier offenbartes Nährmedien vergleichbares Zellwachstum zeigen wie die Kontrolle (Figur 2). Im Unterschied zu Kontrolle produzieren sie weniger Ammonium (Figur 4) und metabolisieren Lactat, während die Kontrolle Lactat produziert (Figur 3). Der pH-Wert der Kultur mit offenbarten Substanzen wandert zu basischen pH-Werten (zu höheren pH-Werten) oder bleibt konstant. Der pH-Wert der Kontrolle (herkömmliches Verfahren) wandert nach saueren pH-Werten (niedriger pH-Wert) (Figur 5).

### Beispiel 7:

Die Standardzelle (Wildtypzelle, die genetisch nicht modifiziert ist) wurde unter Fed-batch-Bedingungen mit folgenden Medienkombination gestestet:

**Tabelle 8: Die Endkonzenration der Substanzen in Nährmedien, die in diesem Experiment eingesetzt worden sind**

| | **Glukose** | **Galaktose** | **Lactat** | **Glutamin** | **Glutamat** | **Asparagin** |
|---|---|---|---|---|---|---|
| | **[g/l]** | **[g/l]** | **[g/l]** | **[g/l]** | **[g/l]** | **[g/l]** |
| | | | | | | |
| **Basalmedium des Experiments S6:St-St-MEL** | 3,4 | 0 | 0 | 0,8 | 0,15 | 0,23 |
| **Feedmedium des Experiments S6:St-St-MEL** | 0 | 35 | Extra feeded | 0 | 5,15 | 6,15 |
| | | | | | | |
| **Basalmedium des Experiments S6:St-MEL-MEL** | 0 | 3 | 2,5 | 0 | 0,8 | 0,23 |
| **Feedmedium des Experiments S6:St-MEL-MEL** | 0 | 35 | Extra feeded | 0 | 5,15 | 6,15 |
| | | | | | | |
| **Basalmedium des Experiments S6:St-MEL-St** | 0 | 3 | 2,5 | 0 | 0,8 | 0,23 |
| **Feedmedium des Experiments S6:St-MEL-St** | 60 | 0 | 0 | 13 | 0,15 | 0,15 |

| | | | | | | |
|---|---|---|---|---|---|---|
| S6:St-St-MEL = Standardzelle (Wildtypzelle), Standard Basalmedium, offenbartes Feedmedium S6:St-MEL-MEL = Standardzelle (Wildtypzelle), offenbartes Basalmedium, offenbartes Feedmedium S6:St-MEL-St = Standardzelle (Wildtypzelle), offenbartes Basalmedium, Standard Feedmedium | | | | | | |

Im Stand der Technik wurde bisher mehrere Kombinationen von Kohlenhydrate in Basalmedien und Feedmedien getestet. Alle Kombinationen sahen vor, dass die Zellen in einem Basalmedium inokuliert werden welches Glukose beinhaltet. Anschließend wurde mit fed-batch Prozess gestartet, in dem das Feedmedium z.B. Galactose beinhaltet. Der Grund für diese Art von Vorgehensweise ist, dass die Zellen in Galaktosehaltigen Basalmedium niedrige Wachstumsrate haben, oder gar nicht wachsen, daher musste man mit glucosehaltigern Basalmedium starten (Altamirano, C., et.al., Biotechnol. Bioeng., 2001, 76(4): 351-60; Altamirano, C., et.al., J. Biotechnology, 2004, 110(2): 171-9; Altamirano, C. et.al., Biotechnol. Progress, 2000, 16(1): 69-75). Gemäß der Erfindung hier aber, haben die Zellen in Galactosehaltigem Medium vergleichbare Wachstumsrate wie in Glucosehaltigem Medium, sodass ein Basalmedium mit erfindungsgemäßen Substanzen völlig neue Möglichkeiten für Zellkultivierung eröffnet. Daher wurde hier ein hier offenbartes Basalmedium mit verschiedenen Feedmedien kombiniert.

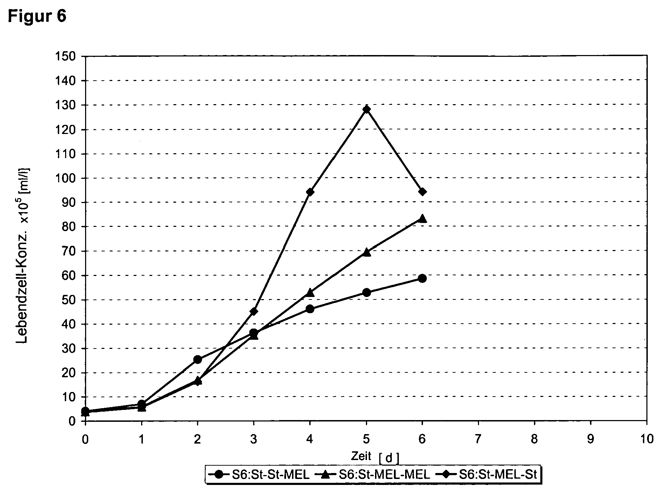

Aus den Daten es ist ersichtlich, dass das offenbarte Feedmedium besser mit erfindungsgemäße Basalmedium kombiniert werden kann (Figur 6, Vergleiche S6:St-St-MEL gegen S6:St-MEL-MEL). Das offenbarte Basalmedium kann man aber auch gut mit einem Standard-Feedmedium kombinieren, in dem das Feedmedium ein anderes Kohlenhydrat beinhaltet als Galaktose, z.B. Glucose. Insbesondere wenn das Basalmedium die hier offenbartes Substanzen beinhaltet und Feedmedium Glucose beinhaltet wachsen die Zellen sogar auf noch höhere Zellkonzentration (Figur 6, vergleiche S6:St-MEL-MEL gegen S6:St-MEL-St).

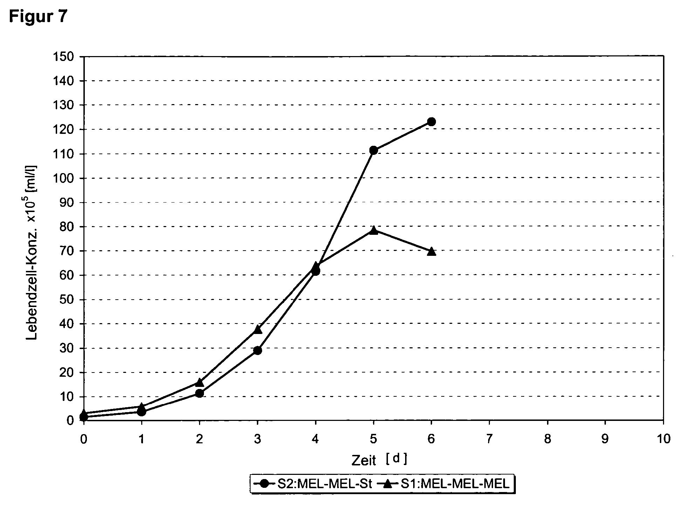

Ein ähnliches Experiment wurde auch mit metabolisch optimierten Zellen durchgeführt (Figur 7). Die Nährmedien des Fed-batch-Experimentes mit metabolisch optimierten Zellen sahen folgender maßen aus:

**Tabelle 9: Die Endkonzentration der Substanzen in Nährmedien, die in diesem Experiment eingesetzt worden sind**

| | **Glukose** | **Galaktose** | **Lactat** | **Glutamin** | **Glutamat** | **Asparagin** |
|---|---|---|---|---|---|---|
| | **[g/l]** | **[g/l]** | **[g/l]** | **[g/l]** | **[g/l]** | **[g/l]** |
| | | | | | | |
| **Basalmedium des Experiments S1:MEL-MEL-MEL** | 0 | 3 | 2,5 | 0 | 0,8 | 0,23 |
| **Feedmedium des Experiments S1:MEL-MEL-MEL** | 0 | 60 | Extra feeded | 0 | 7,15 | 0,15 |
| | | | | | | |
| **Basalmedium des Experiments S2:MEL-MEL-St** | 0 | 3 | 2,5 | 0 | 0,8 | 0,23 |
| **Feedmedium des Experiments S2:MEL-MEL-St** | 60 | 0 | 0 | 13 | 0,15 | 0,15 |

| | | | | | | |
|---|---|---|---|---|---|---|
| S1:MEL-MEL-MEL = Metabolisch optimierte Zelle, offenbartes Basalmedium, offenbartes Feedmedium S2:MEL-MEL-St = Metabolisch optimierte Zelle, offenbartes Basalmedium, Standard Feedmedium | | | | | | |

### Beispiel 9: Fed-batch Experiment mit zwei erfindungsgemäßen Substanzen in Nährmedien

In diesem Experiment wurden 2 erfindungsgemäße Substanzen (Lactat und Succinat) in einem Fed-batch Experiment miteinander kombiniert. Für diese Fed-batch Experimente wurde folgende Nährmedienkombination eingesetzt:

**Tabelle 10: Endkonzentration der eingesetzten Substanzen in Nährmedien dieses Experimentes**

| | **Glukose** | **Galaktose** | **Lactat** | **Glutamin** | **Glutamat** | **Asparagin** | **Na-Succinat** |
|---|---|---|---|---|---|---|---|
| | **[g/l]** | **[g/l]** | **[g/l]** | **[g/l]** | **[g/l]** | **[g/l]** | **[g/l]** |
| | | | | | | | |
| **Basalmedium des Experiments S5:MEL-MEL-MEL** | 0 | 3 | 2 | 0 | 0,8 | 0,23 | 0 |
| **Feedmedium des Experiments S5:MEL-MEL-MEL** | 0 | 35 | Extra feeded | 0 | 5 | 6,15 | 0 |
| | | | | | | | |
| **Basalmedium des Experiments S5:MEL-MEL-MEL-S** | 0 | 3 | 2 | 0 | 0,8 | 0,23 | 0,5 |
| **Feedmedium des Experiments S5:MEL-MEL-MEL-S** | 0 | 35 | Extra feeded | 0 | 5 | 6,15 | 5 |

| | | | | | | | |
|---|---|---|---|---|---|---|---|
| S5:MEL-MEL-MEL = Metabolisch optimierte Zelle, offenbartes Basalmedium mit Lactat, offenbartes Feedmedium mit Lactat S5:MEL-MEL-MEL-S = Metabolisch optimierte Zelle, erfindungsgemäßes Basalmedium mit Lactat und Succinat, erfindungsgemäße Feedmedium mit Lactat und Succinat | | | | | | | |

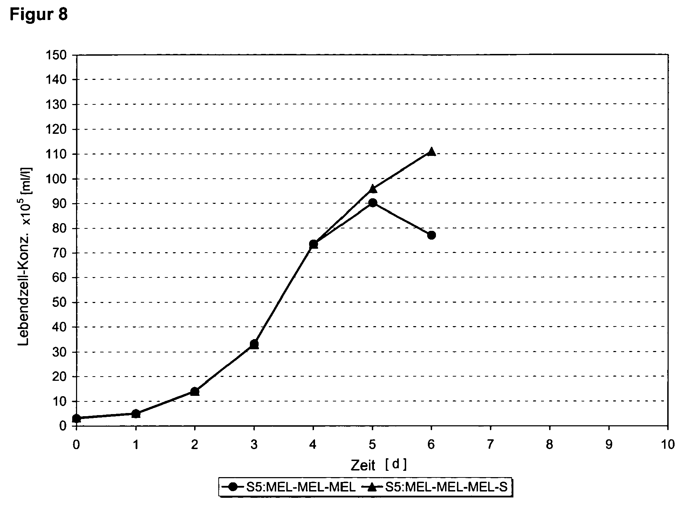

Figur 8 zeigt das Wachstumsverhalten der metabolisch optimierten Zellen in offenbarten Nährmedien mit und ohne Natrium-Succinat.

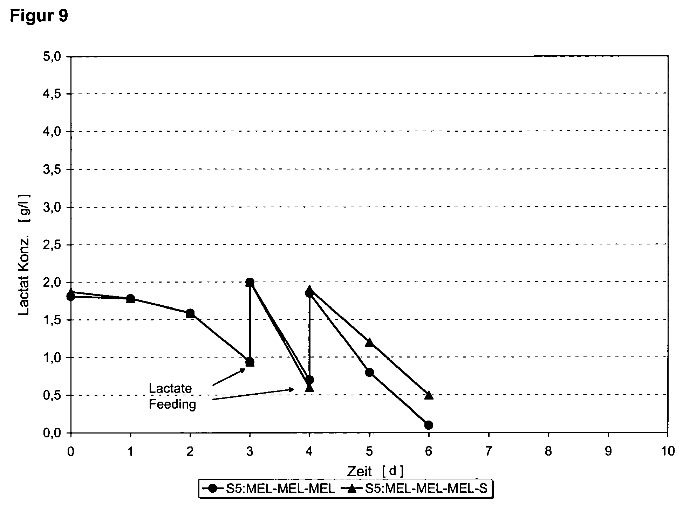

Figur 9 zeigt den Verlauf der Lactatkonzentration in den offenbarten Nährmedien mit und ohne Natrium-Succinat.

## Patentansprüche

1. Verfahren zur Herstellung eines biologischen Produktes, wobei eine Zellkultur, umfassend mindestens eine Säugetier-Zelle aus einer Zelllinie, in einem Nährmedium eingebracht und kultiviert wird und das biologische Produkt aus der Zellkultur und/oder aus dem Nährmedium gewonnen wird, wobei das Nährmedium Bernsteinsäure oder Salze oder Komplexe dieser Säure enthält, wobei das Nährmedium die Bernsteinsäure oder Salze oder Komplexe dieser Säure in einer Konzentration von 0,2 g/l oder größer enthält, wobei das Nährmedium Glutamin in einer Konzentration von kleiner als 8 mmol/l enthält und wobei das Nährmedium Protein-frei ist.

2. Verwendung eines Nährmediums zur Kultivierung einer Zellkultur umfassend mindestens eine Säugetier-Zelle aus einer Zelllinie, wobei das Nährmedium Bernsteinsäure oder Salze oder Komplexe dieser Säure enthält, wobei das Nährmedium die Bernsteinsäure oder Salze oder Komplexe dieser Säure in einer Konzentration von 0,2 g/l oder größer enthält, wobei das Nährmedium Glutamin in einer Konzentration von kleiner als 8 mmol/l enthält und wobei das Nährmedium Protein-frei ist.

3. Verfahren nach Anspruch 1 oder Verwendung nach Anspruch 2, wobei das Nährmedium Glutamin in einer Konzentration von kleiner als 5 mmol/l enthält.

4. Verfahren nach Anspruch 1 oder Verwendung nach Anspruch 2, wobei das Nährmedium Glutamin in einer Konzentration von kleiner als 2 mmol/l enthält.

5. Verfahren nach Anspruch 1 oder Verwendung nach Anspruch 2, wobei das Nährmedium die Bernsteinsäure oder Salze oder Komplexe dieser Säure in einer Konzentration von 1 g/l oder größer enthält.

6. Verfahren nach Anspruch 1 oder Verwendung nach Anspruch 2, wobei das Nährmedium die Bernsteinsäure oder Salze oder Komplexe dieser Säure in einer Konzentration von 5 g/l oder größer enthält.

7. Verfahren nach Anspruch 1 oder Verwendung nach Anspruch 2, wobei das Nährmedium mindestens ein Kohlenhydrat enthält, ausgewählt aus der Gruppe bestehend aus Glucose, Galactose, Fructose, Mannose, Ribose, Glucosamin, Saccharose, Lactose und Mischungen davon.

8. Verfahren oder Verwendung nach Anspruch 7, wobei das Nährmedium das Kohlenhydrat in einer Konzentration von 1 g/l oder größer enthält.

9. Verfahren nach Anspruch 1 oder Verwendung nach Anspruch 2, wobei das Nährmedium frei von Glutamin oder Glutaminderivaten ist.

## Claims

1. Method of production of a biological product, wherein a cell culture, comprising at least one mammalian cell from a cell line, is introduced into a nutrient medium and cultivated and the biological product is obtained from the cell culture and/or from the nutrient medium, wherein the nutrient medium contains succinic acid or salts or complexes of this acid, wherein the nutrient medium contains the succinic acid or salts or complexes of this acid at a concentration of 0.2 g/l or higher, wherein the nutrient medium contains glutamine at a concentration of less than 8 mmol/l and wherein the nutrient medium is protein-free.

2. Use of a nutrient medium for the cultivation of a cell culture comprising at least one mammalian cell from a cell line, wherein the nutrient medium contains succinic acid or salts or complexes of this acid, wherein the nutrient medium the succinic acid or salts or complexes of this acid at a concentration of 0.2 g/l or higher, wherein the nutrient medium contains glutamine at a concentration of less than 8 mmol/l and wherein the nutrient medium is protein-free.

3. Method according to claim 1 or use according to claim 2, wherein the nutrient medium contains glutamine at a concentration of less than 5 mmol/l.

4. Method according to claim 1 or use according to claim 2, wherein the nutrient medium contains glutamine at a concentration of less than 2 mmol/l.

5. Method according to claim 1 or use according to claim 2, wherein the nutrient medium contains the succinic acid or salts or complexes of this acid at a concentration of 1 g/l or higher.

6. Method according to claim 1 or use according to claim 2, wherein the nutrient medium contains the succinic acid or salts or complexes of this acid at a concentration of 5 g/l or higher.

7. Method according to claim 1 or use according to claim 2, wherein the nutrient medium contains at least one carbohydrate, selected from the group consisting of glucose, galactose, fructose, mannose, ribose, glucosamine, sucrose, lactose and mixtures thereof.

8. Method or use according to claim 7, wherein the nutrient medium contains the carbohydrate at a concentration of 1 g/l or higher.

9. Method according to claim 1 or use according to claim 2, wherein the nutrient medium is glutamine-free and is free from glutamine derivatives.

## Revendications

1. Procédé de fabrication d'un produit biologique, sachant qu'une culture cellulaire comprenant au moins une cellule de mammifère d'une lignée cellulaire est disposée dans un milieu nutritif et mise en culture, et que le produit biologique est obtenu à partir de la culture cellulaire et/ou du milieu nutritif, que le milieu nutritif contient de l'acide succinique ou des sels ou complexes de cet acide, que le milieu nutritif contient l'acide succinique ou des sels ou complexes de cet acide dans une concentration de 0,2 g/l ou plus, que le milieu nutritif contient de la glutamine dans une concentration de moins de 8 mmol/l, et que le milieu nutritif est exempt de protéines.

2. Utilisation d'un milieu nutritif pour la mise en culture d'une culture cellulaire comprenant au moins une cellule de mammifère d'une lignée cellulaire, sachant que le milieu nutritif contient de l'acide succinique ou des sels ou complexes de cet acide, que le milieu nutritif contient l'acide succinique ou des sels ou complexes de cet acide dans une concentration de 0,2 g/l ou plus, que le milieu nutritif contient de la glutamine dans une concentration de moins de 8 mmol/l, et que le milieu nutritif est exempt de protéines.

3. Procédé selon la revendication 1 ou utilisation selon la revendication 2, sachant que le milieu nutritif contient de la glutamine dans une concentration de moins de 5 mmol/l.

4. Procédé selon la revendication 1 ou utilisation selon la revendication 2, sachant que le milieu nutritif contient de la glutamine dans une concentration de moins de 2 mmol/l.

5. Procédé selon la revendication 1 ou utilisation selon la revendication 2, sachant que le milieu nutritif contient l'acide succinique ou des sels ou complexes de cet acide dans une concentration de 1 g/l ou plus.

6. Procédé selon la revendication 1 ou utilisation selon la revendication 2, sachant que le milieu nutritif contient l'acide succinique ou des sels ou complexes de cet acide dans une concentration de 5 g/l ou plus.

7. Procédé selon la revendication 1 ou utilisation selon la revendication 2, sachant que le milieu nutritif contient au moins un hydrate de carbone, sélectionné dans le groupe composé de glucose, galactose, fructose, mannose, ribose, glucosamine, saccharose, lactose et de mélanges de ceux-ci.

8. Procédé ou utilisation selon la revendication 7, sachant que le milieu nutritif contient l'hydrate de carbone dans une concentration de 1 g/l ou plus.

9. Procédé selon la revendication 1 ou utilisation selon la revendication 2, sachant que le milieu nutritif est exempt de glutamine ou de dérivés de la glutamine.
